# EUROPEAN PATENT APPLICATION

(11) **EP 2 732 822 A1**
(43) Date of publication of application: **21.05.2014**
(21) Application number: 12815406.9
(22) Date of filing: 13.07.2012
(51) Int. Cl.: A61K 33/00, A61K 47/04, A61K 47/12, A61K 47/32, A61K 47/36, A61P 19/00

(54) **THERAPEUTIC AGENT FOR BONE FRACTURE, BONE GROWTH ENHANCER, OR THERAPEUTIC OR PROPHYLACTIC AGENT FOR BONE DISEASES, EACH COMPRISING CARBON DIOXIDE AS ACTIVE INGREDIENT**

(30) Priority: 15.07.2011 JP 2011156814
(71) Applicant: Neochemir, Inc., Kobe-shi Hyogo 651-0087 (JP); CO2BE Medical Engineering K.K., Hyogo 650-0047 (JP); National University Corporation Kobe University, Kobe-shi, Hyogo 657-8501 (JP)
(72) Inventor: OE, Keisuke, Kobe-shi Hyogo 657-8501 (JP); NIIKURA, Takahiro, Kobe-shi Hyogo 657-8501 (JP); MIWA, Masahiko, Kobe-shi Hyogo 650-0047 (JP); UEHA, Takeshi, Kobe-shi Hyogo 651-0087 (JP); TANAKA, Masaya, Kobe-shi Hyogo 651-0087 (JP)
(74) Representative: Sajda, Wolf E.
(86) International application number: PCT/JP2012/067905
(87) International publication number: WO 2013/011935

(57) **Abstract**

The present invention provides a prophylactic or therapeutic agent for fracture and bone diseases as well as a bone growth promoting agent containing carbon dioxide as an active ingredient. Further, according to the present invention, an additive or synergistic effect for preventing or treating fracture and bone diseases as well as for promoting bone growth by combining the therapeutic agent of the present invention and a bone absorption inhibitor and/or a bone formation promoter is obtained.

## Description

### TECHNICAL FIELD

The present invention is related to a therapeutic agent for bone fracture for prevention and treatment of fracture and bone diseases, and further for promoting bone growth comprising carbon dioxide as an active ingredient. In detail, it is related to a therapeutic or prophylactic agent for bone fracture and bone diseases, and a bone growth promoting agent comprising carbon dioxide as an active ingredient; or a therapy or a prophylaxis for bone fracture and bone diseases and a bone growth promotion of the therapeutic agent of the present invention in combination with a bone resorption inhibitor and/or an osteogenesis promoting agent.

### BACKGROUND ART

Patent Document 1 discloses that carbon dioxide is generally effective for the following symptoms (1) to (10):
(1) itching accompanying mucocutaneous diseases or mucocutaneous disorders such as athlete's foot, insect bite, atopic dermatitis, nummular eczema, xeroderma, seborrheic eczema, urticaria, prurigo, housewives' eczema, acne vulgaris, impetigo, folliculitis, carbuncle, furunculosis, phlegmon, pyoderma, psoriasis, ichthyosis, palmoplantar keratoderma, lichen, pityriasis, wound, burn, rhagades, erosion and chilblain; mucocutaneous injuries such as decubitus ulcer, wound, burn, angular stomatitis, stomatitis, skin ulcer, rhagades, erosion, chilblain and gangrene;
(2) incomplete takes of skin graft, skin flap, etc.;
(3) dental diseases such as gingivitis, alveolar pyorrhea, denture ulcer, nigricans gingiva, and stomatitis;
(4) skin ulcers, cryesthesia and numbness caused by peripheral circulatory disorders such as thromboangitis obliterans, arteriolosclerosis obliterans, diabetic peripheral circulatory disorder, and lower limb varicosis;
(5) musculoskeletal diseases such as chronic rheumatoid arthritis, cervico-omo-brachial syndrome, myalgia, arthralgia and lumbago;
(6) nervous system diseases such as neuralgia, polyarthritis and subacute myelo-optic neuropathy;
(7) keratoses such as psoriasis, corns, calluses, ichthyosis, palmoplantar keratoderma, lichen, and pityriasis;
(8) suppurative skin diseases such as acne vulgaris, impetigo, folliculitis, carbuncles, furuncules, phlegmon, pyoderma, and suppurative eczema ;
(9) constipation caused by loss of reflection of defecation; and
(10) suppression of hair regrowth after depilation (treatment of unwanted hair); cosmetic troubles with the skin or hair such as freckles, rough skin, muddy complexion, faded skin complexion and loss of hair glossiness, and partial obesity.

In order to achieve an improvement of the above-mentioned symptoms, as a carbon dioxide supplying means, a composition for preparing a carbon dioxide for external use (see Patent Documents 2 and 3), a carbon dioxide composition for external use (see Patent Document 4), a composition for preparing a carbon dioxide gel for external use (see Patent Document 5), a material for preparing a carbon dioxide agent for external use (see Patent Document 6), and a carbon dioxide external administration device (see Patent Documents 7 and 8) are disclosed.

Also use of the mean for absorbing carbon dioxide for strengthening muscle is disclosed (Patent Document 9).

However, a therapeutic agent for bone fracture, a bone growth promoting agent, or a therapeutic or prophylactic agent for bone diseases containing carbon dioxide as an active ingredient has never been known.

In a healthy subject, the amount and the strength of a bone tissue are maintained by a dynamic equilibrium between bone resorption and osteogenesis. It is considered that bone mass is decreased and a bone is easily fractured when osteogenesis is reduced or bone resorption is enhanced when the equilibrium is disturbed by aging and/or irregular hormone balance. In order to improve such condition, one or more of calcium, activated vitamin D, calcitonin, estrogen, bisphosphonate and the like is used in a medicinal therapy, but these agents have no activity to increase bone mass or regenerate bone.

At present, growth factors such as BMP (Bone Morphogenetic Protein) or FGF (Fibroblast Growth Factor) etc. are studied as a substance for promoting osteogenesis, but these factors are protein and there are problems like necessity of injection to administrate, low stability in vivo and/or expensiveness.

### PRIOR ART REFERENCES

### PATENT DOCUMENT

Patent Document 1: JP 2000-319 87 A
Patent Document 2 WO 2002/80941 A
Patent Document 3 WO 2006/80398 A
Patent Document 4 WO 2003/57228 A
Patent Document 5 WO 2005/16290 A
Patent Document 6 WO 2004/04745 A
Patent Document 7 WO 2004/02393 A
Patent Document 8 WO 007/112726 A
Patent Document 9 JP 2009-120 606 A

### DISCLOSURE OF INVENTION

The object of the present invention is to provide therapeutic agent for bone fracture for prevention and treatment of bone fracture and bone diseases, and further for enhancing bone growth , the agent comprising carbon dioxide as an active ingredient, which is convenient, safe and inexpensive.

### MEANS TO SOLVE THE PROBLEM

Inventors of the present invention have completed the present invention by realizing that bone growth is enhanced and the period for healing of bone fracture is reduced by absorption of carbon dioxide into the body.

The present invention is a therapeutic agent for bone fracture, a bone growth promoting agent and a therapeutic and prophylactic agent for bone diseases comprising carbon dioxide as an active ingredient.

The present invention is a therapeutic agent for bone fracture, a bone growth promoting agent and a therapeutic and prophylactic agent for bone diseases, in which carbon dioxide is locally administrated.

The present invention is a therapeutic agent for bone fracture, a bone growth promoting agent and a therapeutic and prophylactic agent for bone diseases comprising carbon dioxide as an active ingredient, in which carbon dioxide is transdermally administrated through the skin close to the target site.

The present invention is a therapeutic agent for bone fracture, a bone growth promoting agent and a therapeutic and prophylactic agent for bone diseases comprising carbon dioxide as an active ingredient, in which carbon dioxide is transdermally administrated through the skin far from the target site.

The following embodiments are preferable in this invention.

A preferable example of the present invention is a therapeutic agent for bone fracture, a bone growth promoting agent and a therapeutic and prophylactic agent for bone diseases, wherein means for absorbing carbon dioxide are provided which means is a carbon dioxide external administration device.

A preferable example of the present invention is a therapeutic agent for bone fracture, a bone growth promoting agent and a therapeutic and prophylactic agent for bone diseases, wherein means for absorbing carbon dioxide are provided which means is a carbon dioxide external administration device, including a sealing enclosure member capable of sealing off a body surface of a human or an animal from outside air; a supply unit for supplying carbon dioxide to inside of the sealing enclosure member and an absorption aid that assists transdermal absorption of carbon dioxide, which includes a medium to dissolve carbon dioxide, inside of the sealing enclosure member above.

A preferable example of the present invention is a therapeutic agent for bone fracture, a bone growth promoting agent and a therapeutic and prophylactic agent for bone diseases comprising carbon dioxide as an active ingredient, wherein means are provided for absorbing carbon dioxide which means is a carbon dioxide external administration device, including a sealing enclosure member capable of sealing off a body surface from outside air; a supply unit for supplying carbon dioxide into the inside of the sealing enclosure member, and perspiration promoting means for promoting perspiration at the body surface inside the sealing enclosure member.

A preferable example of the present invention is a therapeutic agent for bone fracture, a bone growth promoting agent and a therapeutic and prophylactic agent for bone diseases comprising carbon dioxide as an active ingredient, wherein the agent(s) is(are) a carbon dioxide external preparation which is(are) one or more selected from a group consisting of:
(1) a carbon dioxide external agent including a medium which dissolves carbon dioxide and carbon dioxide is dissolved therein;
(2) a carbon dioxide external agent consisting of a solid material containing an acid, and a liquid material containing a carbonate salt, the preparation being obtained by mixing the granular material above and the liquid material above at use;
(3) a carbon dioxide external agent consisting of a solid material containing a carbonate salt, and a liquid material containing an acid, the preparation being obtained by mixing the granular material above and the liquid material above at use;
(4) a carbon dioxide external agent obtained by adding water to a solid material containing an acid and a carbonate salt at use;
(5) a carbon dioxide external agent consisting of a liquid material containing an acid, and a liquid material containing a carbonate salt, which is obtained by mixing these liquid materials at use;
(6) a carbon dioxide external agent obtained from a composition for preparing an external carbon dioxide agent, comprising a granular material containing a water-soluble acid, a thickener and a water-soluble dispersant as the essential components wherein said thickener is mixed with the water-soluble acid and the water-soluble dispersant; and a viscous material containing a carbonate, water and a thickener as the essential components, which is to be mixed with the granular material at use;
(7) a carbon dioxide external agent obtained from a composition for preparing carbon dioxide gel for external use characterized by comprising granular material (A) and viscous material (B) to be mixed with the granular material (A):
   (A) a granular material comprising a weak acid and a calcium ion trapping agent as essential components;
   (B) a viscous material comprising calcium carbonate, a gelling agent to be gelated with calcium ions and water as essential components,
(8) a carbon dioxide external agent obtained from a composition for preparing a carbon dioxide external agent characterized by comprising a substance generating an acid after being hydrolyzed, a carbonate, a thickener, and water as essential components,
(9) a composition of carbon dioxide for external use (synonymous with carbon dioxide external agent) characterized by a viscous material comprising water and a thickener at least, in which carbon dioxide is dissolved substantially in a non-bubble state; or a composition of carbon dioxide for external use (synonymous with carbon dioxide external agent) comprising a fermenting micro-organism and metabolite thereof, a thickener, water and carbon dioxide at least, and
(10) a carbon dioxide external agent obtained from a material for formation of carbon dioxide external preparation characterized by comprising a base agent that comprises a polymeric three-dimensional network structure impregnated with a viscous material containing at least an acid and water, and is made to contact with skin at use, and a reactant that contains at least a carbonate, and is made to contact with the base agent at use so as to generate carbon dioxide.

A preferable example of the present invention is a therapeutic agent for bone fracture, a bone growth promoting agent and a therapeutic and prophylactic agent for bone diseases, wherein carbon dioxide is transdermally absorbed by taking a bath in a carbonated spring.

A preferable example of the present invention is a therapeutic agent for bone fracture, a bone growth promoting agent and a therapeutic and prophylactic agent for bone diseases, wherein the concentration of carbon dioxide is equal to or over 300 ppm.

A preferable example of the present invention is a therapeutic agent for bone fracture, a bone growth promoting agent and a therapeutic and prophylactic agent for bone diseases, which is injected into the tissue of the target site.

A preferable example of the present invention is a therapeutic agent for bone fracture, a bone growth promoting agent and a therapeutic and prophylactic agent for bone diseases, wherein a means of supplying carbon dioxide is a local administration of a mixture of carbon dioxide and vapor of a medium in which carbon dioxide can be dissolved.

### EFFECT OF THE INVENTION

A therapeutic agent for bone fracture of the present invention comprising carbon dioxide as an active ingredient can reduce the period for healing of bone fracture easily and with low cost, and further prevent pseudoarthrosis etc. It is possible to prevent or treat a bone disease such as osteoporosis and rheumatoid arthritis etc. Also it is possible to increase height or form a bone which is hardly fractured by enhancing bone growth, when the agent is used for a child or a young animal in the period in growth. Further it is possible to prevent a decrease of bone under the microgravity environment in a space station etc. easily and in safe.

### BRIEF DESCRIPTION OF THE DRAWINGS

- FIG. 1: A ratio of the length between the right tibia and the left tibia in the carbon dioxide absorption group is shown on the assumption that the corresponding ratio in a control group was 1.
- FIG. 2: PCNA staining of tibia auxotonic gristle plate in the carbon dioxide FIG. 3 absorption group and the control group is shown. Each length of the tibia in the control group, the training group and the carbon dioxide absorption group is shown.
- FIG. 4: Each length of the tibia auxotonic gristle plate in the control group, the training group and the carbon dioxide absorption group is shown.
- FIG. 5: is a skeleton framework of a carbon dioxide external administration device used in the present invention.
- FIG. 6: is a skeleton framework of a carbon dioxide external administration device used in the present invention.
- FIG. 7: is a skeleton framework of a carbon dioxide external administration device used in the present invention.
- FIG. 8: is a skeleton framework of a carbon dioxide external administration device used in the present invention.
- FIG. 9: is a perspective view of a sealing enclosure member which can be used in the present invention.
- FIG. 10: is a skeleton framework of a carbon dioxide external administration device used in the present invention.
- FIG. 11: is a skeleton framework of a carbon dioxide external administration device used in the present invention.
- FIG. 12: is a skeleton framework of a carbon dioxide external administration device (the first embodiment) used in the present invention.
- FIG. 13: is a skeleton framework of a carbon dioxide external administration device (the second embodiment) used in the present invention.
- FIG. 14: is a skeleton framework of a carbon dioxide external administration device (the third embodiment) used in the present invention.
- FIG. 15: is a skeleton framework of a carbon dioxide external administration device (the fourth embodiment) used in the present invention.
- FIG. 16: is a skeleton framework of a carbon dioxide external administration device (the fifth embodiment) used in the present invention.
- FIG. 17: is a skeleton framework of a carbon dioxide external administration device (the sixth embodiment) used in the present invention.

### DISCLOSURE OF INVENTION

There is no limitation in the therapeutic agent for bone fracture, a bone growth promoting agent and a therapeutic and prophylactic agent for bone diseases of the present invention comprising carbon dioxide as an active ingredient, as long as carbon dioxide is absorbed into the body. Examples of the means for absorption of carbon dioxide into the body include the followings:
(1) a carbon dioxide external agent prepared from a composition for preparing carbon dioxide external agent such as (a) a composition for preparing a carbon dioxide external agent characterized by comprising a substance generating an acid after being hydrolyzed, a carbonate, a thickener, and water as essential components, and further a gelating agent gelated by calcium ion, and a water-insoluble or poorly soluble calcium salt (WO 2006/80398); or (b) a composition for preparing an external carbon dioxide agent comprising a granular material containing a water-soluble acid, a thickener and a water-soluble dispersant as the essential components wherein the said thickener is mixed with the water-soluble acid and the water-soluble dispersant; and a carbonate, water and a thickener as the essential components, which are to be mixed with the said granular material at use (WO 2002/80941);
(2) a composition of carbon dioxide for external use characterized in that carbon dioxide is dissolved in a viscous material comprising water and a thickener substantially in a non-bubble state, or characterized in comprising a fermenting micro-organism and metabolite thereof, a thickener, water and carbon dioxide at least(WO 2003/57228);
(3) a carbon dioxide external agent obtained from a composition for preparing carbon dioxide gel for external use characterized by comprising granular material (A) and viscous material (B) to be mixed with the granular material (A):
   (A) a granular material comprising a weak acid and a calcium ion trapping agent as essential components;
   (B) a viscous material comprising calcium carbonate, a gelling agent to be gelated with calcium ions and water as essential components(WO 2005/16290);
(4) a carbon dioxide external administration device, comprising a sealing enclosure member capable of sealing a body surface from ambient air, supply unit for supplying carbon dioxide into the inside of the sealing enclosure member, and an absorption aid capable of assisting the transdermal or transmucosal absorption of carbon dioxide inside the sealing enclosure member (WO 2004/02393); and
(5) a carbon dioxide external administration device characterized by including: a sealing enclosure member capable of sealing the body surface of a human or an animal from the outside air; a supply unit for supplying carbon dioxide into the inside of the sealing enclosure member; and perspiration promoting means for promoting perspiration at the body surface inside the sealing enclosure member (WO 2008/047829).

In addition, water of carbonated spring including an artificial carbonated spring, and carbon dioxide-containing water vapor are also available. The carbon dioxide-containing water vapor means vapor such as natural carbon dioxide-containing water vapor which bursts from the ground, which is used for treating hemorrhoid etc. in places like Eastern Europe; and white smoke of carbon dioxide-containing water vapor which is generated by putting dry-ice in the air. A method of injecting gaseous carbon dioxide into the target tissue directly using an injection needle can also be available.

Of course, it is not intended that the present invention is limited to these means for supplying carbon dioxide and any means which enable carbon dioxide to be absorbed into the targeted part in an amount required for preventing or treating fracture and a bone disease, or enhancing a bone growth. Among them, the above mentioned carbon dioxide external administration device (4) or (5) is more preferable.

Embodiments of the present invention when the two carbon dioxide external administration devices (4) and (5) described above are explained below with reference to the drawings. In addition, the same sign means the same element or corresponding element in each drawing.

### A. Use of a carbon dioxide external administration device (4)

The carbon dioxide external administration device 1 is constituted from at least a sealing enclosure member 2 capable of sealing a body surface from the outside air, a supply means 3 for supplying carbon dioxide into the inside of the sealing enclosure member 2, and an absorption aid 4 that is adapted to assist transdermal or transmucosal absorption of the carbon dioxide inside the sealing enclosure member 2. Note, however, that the sealing enclosure member 2 does not necessarily have to seal the body surface from the outside air completely, but rather it is sufficient to so long as the carbon dioxide concentration inside the sealing enclosure member 2 can be maintained above a certain level by continuously supplying a small amount of carbon dioxide into the sealing enclosure member 2.

Also there are no particular limitations on the shape, materials and so on of the sealing enclosure member 2, so long as the sealing enclosure member 2 covers the skin or mucosa and has a space therein, in which carbon dioxide is held.

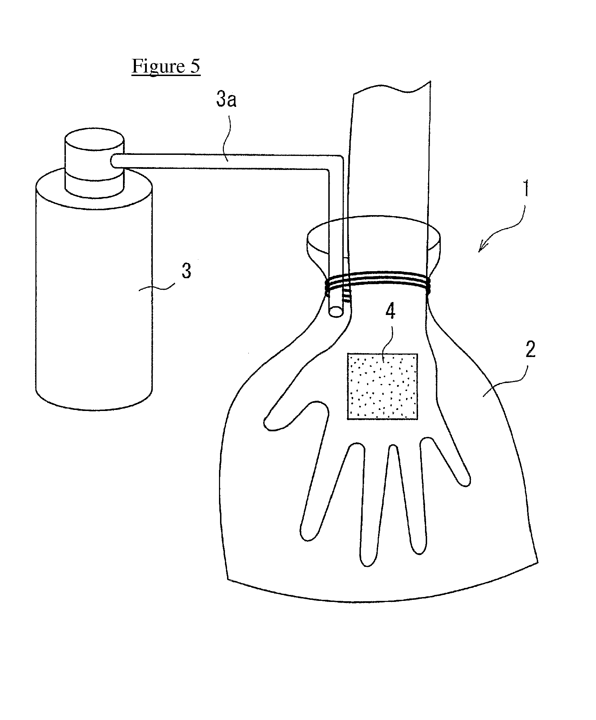

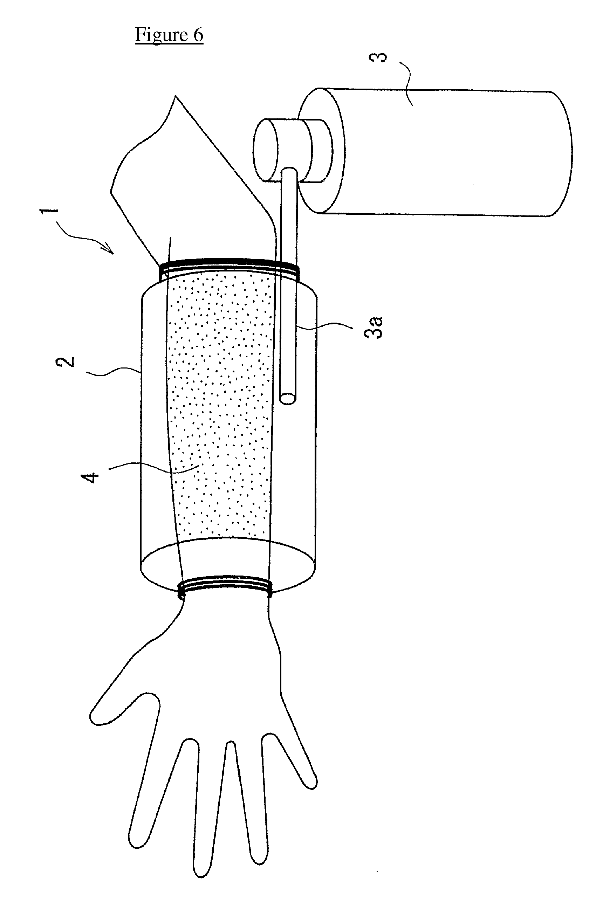

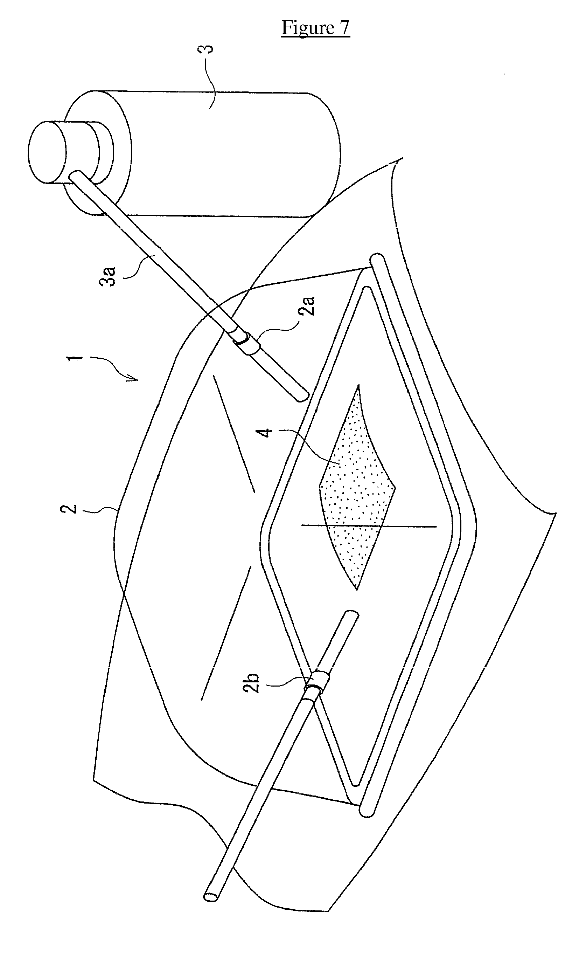

Regarding the shape, for example, a bag that can completely pack a hand (or a foot) as shown in FIG. 5, and a tubular bag that wraps around an arm (or a leg) as shown in FIG. 6 can be used. Moreover, as the shape of the sealing enclosure member 2, a vessel having an open hemline that adheres to a relatively large area of the skin surface such as the abdomen or the like as shown in FIG. 7, or a cup-shaped tubular body that has one end thereof open and the other end thereof made to be a part connecting to the supply means 3 as shown in Fig .8 can be used. Of course, the shape of the sealing enclosure member 2 is not limited to these examples.

There are no particular limitations on the materials of the sealing enclosure member 2 so long as these materials are impermeable to gases; materials can be selected from metal, plastic, rubber, glass and so on and used as appropriate in accordance with the purpose, the part of application and so on.

In the case that the sealing enclosure member 2 is one made of only a non-elastic and hard material such as metal or glass (hereinafter referred to as "rigid sealing enclosure member"), a shape having therein a certain sealed space surrounding the part of application is preferable. The part contacting with the skin or mucosa preferably has a shape conforming to the part of application so that carbon dioxide will not leak out; combining with an elastic material such as rubber or a resin for the part contacting with the skin or mucosa is preferable since it will be possible to flexibly respond to the part of application The combination with a material that has higher flexibility and excellent adhesion to the skin or mucosa such as a viscoelastic gel is more preferable.

Moreover, to fill carbon dioxide into the sealed space in which the skin or mucosa is sealed by the rigid sealing enclosure member, the enclosure member preferably has a gas outlet therein. When injecting carbon dioxide into the sealed space, air in the sealed space escapes out from a gap in the part contacting with the skin or mucosa and the air is replaced with carbon dioxide.

But the adjustment of the size of the gap is so hard that it is difficult to carry out the replacement reliably, and the amount of carbon dioxide required for the replacement is not fixed. For example, by providing a gas outlet in, a position furthest from the gas inlet, the replacement of the air with carbon dioxide can be carried out efficiently in a short period of time.

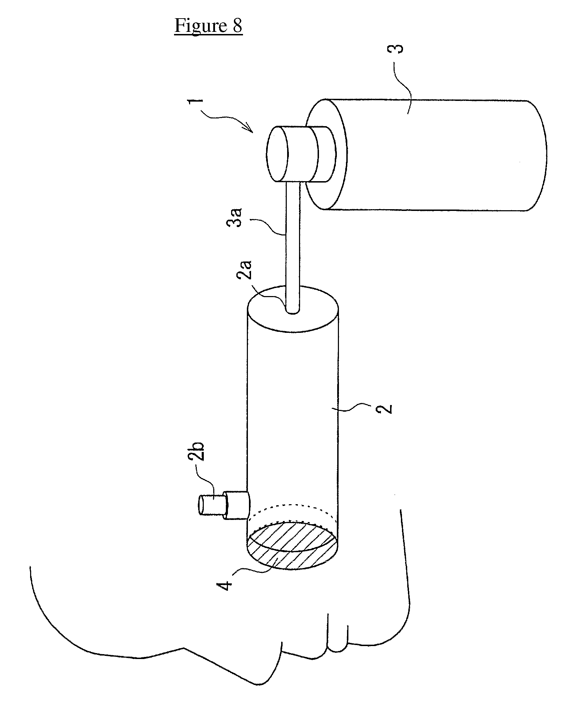

The gas inlet 2a in the rigid sealing enclosure member (see FIGs. 7 and 8) has connected thereto a tube 3a that is connected to the supply means 3; there are no particular limitations on the gas inlet 2a so long as gas will not leak therefrom, but one equipped with a check valve for preventing back flow of gas is more preferable. Regarding the gas outlet 2b in the rigid sealing enclosure member (see FIG. 7 and FIG. 8), a gap in the part contacting with the skin or mucosa may be used, but to carry out the replacement of the air inside the enclosure member with carbon dioxide reliably and efficiently, a gas outlet equipped with a check valve for preventing back flow of gas is more preferable.

In the case that the sealing enclosure member 2 is one made of a material that is a flexible material such as a rubber, a resin or a soft plastic, especially, a material that has a shape holding ability, by which the shape is barely kept by some external force (hereinafter referred to as a "flexible sealing enclosure member"), the part contacting with the skin or mucosa is also flexible, and hence the above mentioned material can be used as is.

However, the combination with a material that has yet higher flexibility and excellent adhesion to the skin or mucosa such as a viscoelastic gel for the part contacting with the skin or mucosa is more preferable. Moreover, with such a flexible sealing enclosure member, the same gas inlet and gas outlet as those in the case of a rigid sealing enclosure member can be used.

In the case that the sealing enclosure member 2 is one made of a material that is a flexible material such as a rubber or a resin, especially a material that is elastic highly like a balloon (hereinafter referred to as an "elastic sealing enclosure member"), this can be used in the form of a tube, a bag or the like, with the skin or mucosa being sealed by binding the mouth of the tube, bag or the like covering the desired part, or providing a retractable opening/closing mouth in which rubber, a spring or the like is placed in the mouth.

Moreover, in the case of a tubular elastic sealing enclosure member for which the part of application is made to be, for example, an arm, the circumference of the tube may be made to be approximately the same as that of the arm, or may be made smaller, with the skin or mucosa being sealed in a state with the sealing enclosure member adhering to the skin or mucosa, or a state with only a very small space between the sealing enclosure member and the skin or mucosa; carbon dioxide may then be injected between the sealing enclosure member and the skin or mucosa so as to expand the sealing enclosure member, and the certain space in which carbon dioxide may be held is created by the carbon dioxide itself.

A gas inlet and gas outlet of the elastic sealing enclosure member may have the same structure as with the aforementioned rigid sealing enclosure member, but alternatively it is possible to seal the skin or mucosa with a tube, a bag or the like, and discharge the air therein as much as possible in advance, and then insert the tube 3a in from the orifice of the elastic sealing enclosure member having the form of a tube, a bag or the like such that air is prevented from entering therein as much as possible, and inject carbon dioxide (see FIGs. 5 and 6).

In the case that the sealing enclosure member 2 is one made of a foldable sheet- or film-like material (hereinafter referred to as a "sheet-type sealing enclosure member"), this can be used in the form of a tube, a bag or the like, with the skin or mucosa being sealed by binding the orifice of the tube, the bag or the like covering the desired part, or providing a retractable opening/closing orifice in which rubber, a spring or the like is placed in the orifice.

Moreover, in the case of a tubular sheet-type sealing enclosure member for which the part of application is made to be, for example, an arm (or a leg), it is possible to make the circumference of the tube to be greater than that of the arm, seal the arm and fold the sealing enclosure member, so as to discharge air therein as much as possible in advance, and then use the sealing enclosure member with adhering to the skin or mucosa.

A gas inlet and gas outlet of the sealing enclosure member may be as with the rigid sealing enclosure member, but alternatively it is possible to seal the skin or mucosa with the tube, bag or the like, and discharge the air therein as much as possible in advance, and then insert a tube in from the orifice of the sheet-type sealing enclosure member having the form of a tube, a bag or the like such that air is prevented from entering therein as much as possible, and inject carbon dioxide.

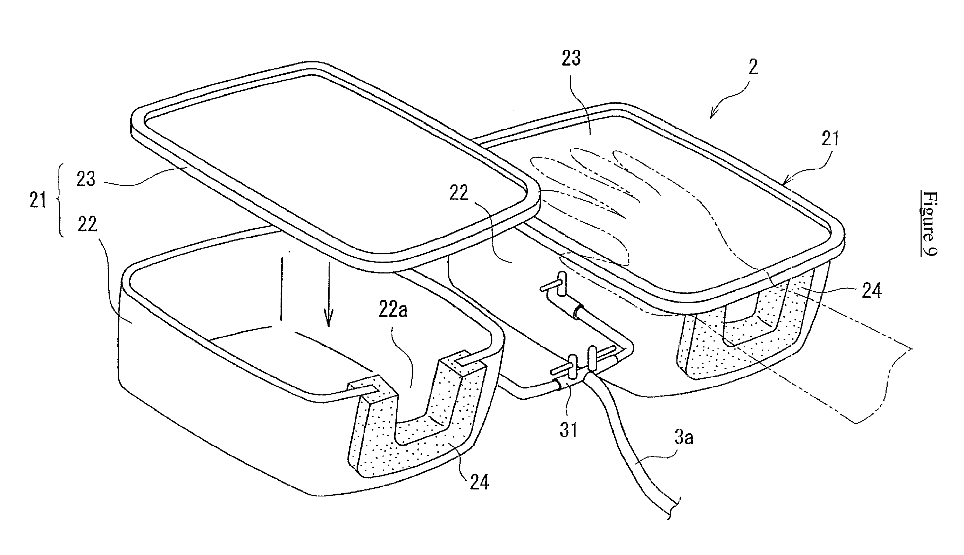

FIG. 9 shows an example of sealing enclosure members 2 that can be suitably used in the present invention.

These sealing enclosure members 2 comprise a right and left pair of storage boxes 21 made of a transparent or translucent synthetic resin; a tube 3a possessing a valve 31 is connected to the storage boxes 21, whereby carbon dioxide from the supply means 3 can be supplied to either or both of the storage boxes 21.

Each storage box 21 comprises a box body 22 that is open at the top and in a side wall thereof a cut-out concave portion 22a, through which a wrist can be inserted, is formed, and a detachable lid member 23 that covers the upper opening of the box body 22; a sealing member 24 made of a flexible material such as sponge is attached around the rim of the cut-out concave portion 22a. Moreover, although not shown in FIG. 9, a sealing member made of a flexible material such as sponge is also attached to the place corresponding to the cut-out concave portion 22a of the rim of the rear surface of the lid member 23.

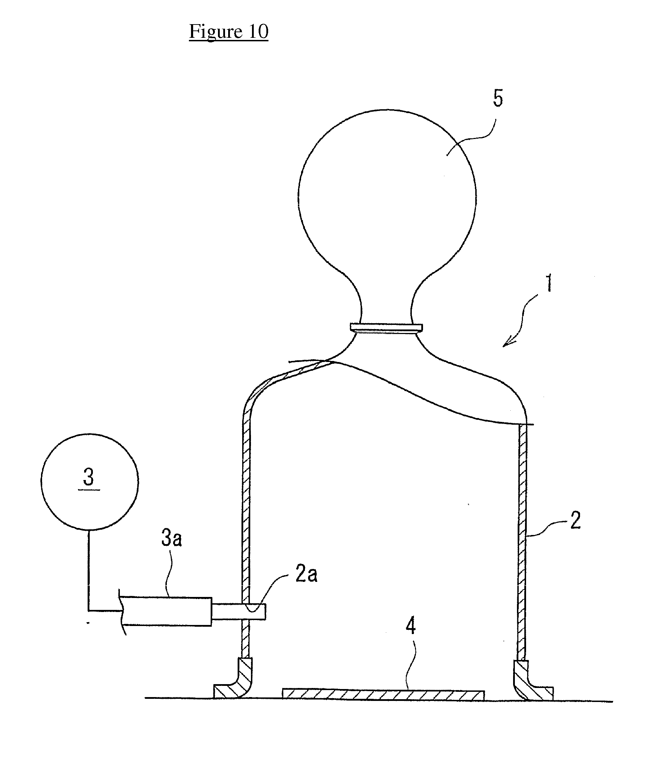

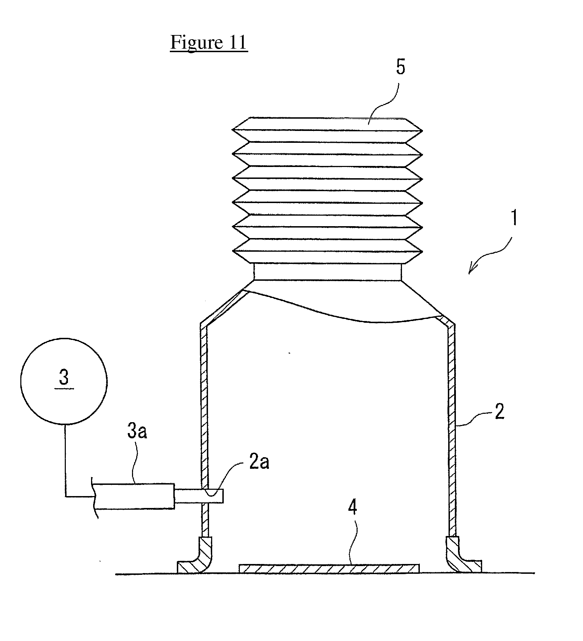

FIGs. 10 and 11 show carbon dioxide external administration devices 1 according to other embodiments of the present invention.

With each of the administration devices 1 in this case, a carbon dioxide amount indicator 5 that expands upon carbon dioxide being supplied into the sealing enclosure member 2 and contracts by the decrease of carbon dioxide is attached to an upper end of the sealing enclosure member 2, whereby transdermal or transmucosal absorption of carbon dioxide can easily be checked upon from the outside even without specialized knowledge or the like.

Moreover, of the administration devices 1 shown in FIGs. 10 and 11, with the administration device 1 shown in Fig 10, a balloon indicator 5 made of rubber or another elastic material is used, and with the administration device 1 shown in FIG. 11, a bellows-structured indicator 5 that is retractable in a vertical direction is used.

With the carbon dioxide external administration device 1 of the present invention, there are no particular limitations on the carbon dioxide supply means 3; for example, a commercially available carbon dioxide gas cylinder or the like can be used. Moreover, there may be used, for example, a device that uses a closed vessel having a tube attached thereto, wherein so-called dry ice, which is solid carbon dioxide, is vaporized inside the vessel, or carbon dioxide is generated inside the vessel through reaction between a carbonate and an acid, and the like.

With the carbon dioxide external administration device 1 of the present invention, regarding the tube 3a connecting the sealing enclosure member 2 to the carbon dioxide supply means 3, there are no particular limitations so long as the tube is such that gas will not leak out, and any material from which a tube can be formed, for example, rubber, a resin, metal or glass, may be used.

With the carbon dioxide external administration device 1 of the present invention, there are no particular limitations on a sheet-like product used for the carbon dioxide absorption aid 4, so long as this sheet-like product can be impregnated with a liquid containing at least water and can be patched on skin or mucosae; examples thereof include woven and nonwoven cloths made of natural fibers, synthetic fibers, semi-synthetic fibers or the like, semi-permeable membranes such as cellulose membranes, and hydrogel sheets made of a natural polymer, a synthetic polymer, a semi-synthetic polymer or the like, and one or more of these may be used.

There are no particular limitations on the aforementioned liquid containing at least water; this liquid may be water itself, or so long as the effects of the present invention are not impaired, may be water having some substances dissolved or dispersed therein.

Moreover, the liquid containing at least water preferably has a pH value of 7 to 2, and more preferably is acidic water having a pH value of 6.5 to 4. The reason is that when carbon dioxide is dissolved in an acidic solvent of pH value of not less than 4, the carbon dioxide is transdermally or transmucosally absorbed efficiently.

With the carbon dioxide external administration device 1 of the present invention, a viscous material used for the carbon dioxide absorption aid 4 may be liquid or semi-solid, so long as the viscous material contains water and does not easily run off or after having been applied onto the skin or mucosae, and has a viscosity of not less than 20 cps at 20 °C; examples of forms include liquids, creams, pastes and gels; and one or more of these may be used.

Moreover, the viscous material preferably has a pH value of 7 to 2, and more preferably is an acidic viscous material having a pH value of 6.5 to 4.

There are no particular limitations on the viscous material, but the water content is preferably as high as possible, and moreover it is preferable to be one that is able to replenish moisture in the skin or mucosae. As a liquid, an aqueous solution, suspension, swelling liquid or the like of a thickener may be suitably used, because the required viscosity can be obtained with a small amount, and adhesion or viscosity to skin or mucosae is excellent. There are no particular limitations on the production method thereof, with it being possible to use a known method.

There are no particular limitations on a cream, a paste or a gel; one produced using a known method can be used.

As the thickener used in the viscous material, one or more selected from the group consisting of natural polymers, semi-synthetic polymers, synthetic polymers and inorganic materials can be used.

Examples of natural polymers include plant-derived polymers such as gum arabic, carrageenan, galactan, agar, xanthan gum, quince seed gum, guar gum, tragacanth, pectin, mannan, locust bean gum, wheat starch, rice starch, corn starch and potato starch; microorganism-derived polymers such as curdlan, xanthan gum, succinoglucan, dextran, hyaluronic acid and pullulan; and protein-type polymers such as albumin, casein, collagen, gelatin and fibroin; and one or more of these can be used. Among them, from the standpoint of affinity with skin and mucosae and so on, gum arabic, carrageenan, xanthan gum, tragacanth, hyaluronic acid, pullulan, pectin, mannan and locust bean gum can be preferably used, and from the standpoint of feeling in use and so on, carrageenan and pectin can be more preferably used.

Examples of semi-synthetic polymers include cellulosic polymers such as ethylcellulose, carboxymethylcellulose, carboxymethylethylcellulose, carboxymethyl starch, croscarmellose, crystalline cellulose, cellulose acetate, cellulose acetate phthalate, hydroxyethylcellulose, hydroxypropylcellulose, hydroxypropylmethylcellulose, hydroxypropylmethylcellulose phthalate, methylcellulose and methylhydroxypropylcellulose; starch-type polymers such as pregelatinized starch, partially pregelatinized starch, carboxymethyl starch, dextrin, methyl starch, starch-acrylic acid copolymers and cellulose-acrylonitrile graft copolymers; alginate-type polymers such as sodium alginate and propylene glycol alginate, and other polysaccharide-type polymers such as chondroitin sulfate sodium and sodium hyaluronate; and one or more of these can be used.

Among them, from the standpoint of affinity with skin and mucosae and so on, sodium alginate, propylene glycol alginate, sodium carboxymethylcellulose, dextrin and sodium hyaluronate can be preferably used, and from the standpoint of feeling in use and so on, sodium alginate, propylene glycol alginate and sodium hyaluronate can be more preferably used.

Examples of synthetic polymers include carboxyvinyl polymers, sodium polyacrylate, polyamines, polyacrylamide, polyvinylacetal diethylaminoacetate, polyvinyl alcohol, polyvinyl pyrrolidone, polyethylene glycol, polyethylene oxide, poly(meth)acrylic acid, polyvinyl methyl ether and so on; one or more of these can be used. Among them, from the standpoint of affinity with skin and mucosae and so on, carboxyvinyl polymers, sodium polyacrylate, polyacrylamide, polyvinyl alcohol and polyvinyl pyrrolidone can be preferably used, and from the standpoint of feeling in use and so on, polyvinyl alcohol, polyvinyl pyrrolidone and carboxyvinyl polymers can be more preferably used.

Examples of inorganic materials include hydrated silicon dioxide, light anhydrous silicic acid, colloidal alumina, bentonite, laponite and so on; and one or more of these can be used.

With the carbon dioxide external administration device 1, there are no particular limitations on an alcohol having a high vaporization temperature used in the carbon dioxide absorption aid 4, so long as this alcohol is liquid or semi-solid at room temperature and does not readily vaporize at the skin temperature of a human; examples thereof include monohydric alcohols such as isopropyl alcohol and 1-butanol, and polyhydric alcohols such as ethylene glycol, diethylene glycol, polyethylene glycol, propylene glycol, dipropylene glycol, 1,3-butanediol, 1,2-pentanediol, isoprene glycol, glycerol, diglycerol, triglycerol and tetraglycerol; and one or more of these can be used.

There are no particular limitations on the method of using the alcohol; in accordance with the physical properties thereof and so on, the alcohol may be applied or sprayed onto the skin or mucosa as is, or impregnated or the like into a nonwoven cloth or the like and then patched on the skin.

With the carbon dioxide external administration device 1, there are no particular limitations on oil and fat used in the carbon dioxide absorption aid 4, so long as the oil and fat is liquid or semi-solid at room temperature and can be applied relatively thinly onto the skin or mucosa; examples thereof include natural vegetable oils and fats such as avocado oil, avocado butter, olive oil, sesame oil, safflower oil, soybean oil, camellia oil, sunflower oil and macadamia nut oil, and animal oils and fats such as squalane, mink oil, beef tallow, lard, chicken fat and horse fat; and one or more of these can be used.

There are no particular limitations on the method of using oil and fat; in accordance with the physical properties thereof and so on, the oil and fat may be applied or sprayed onto the skin or mucosa as is, or impregnated or the like into a nonwoven cloth or the like and then patched on the skin.

With the carbon dioxide external administration device 1, there are no particular limitations on a wax used in the carbon dioxide absorption aid 4, so long as the wax is liquid or semi-solid at room temperature and can be applied relatively thinly onto the skin or mucosa; examples thereof include jojoba oil, carnauba wax, candelilla wax, beeswax and lanolin; and one or more of these can be used.

There are no particular limitations on the method of using the wax; in accordance with the physical properties thereof and so on, the wax may be applied or sprayed onto the skin or mucosa as is, or impregnated or the like into a nonwoven cloth or the like and then patched on the skin.

With the carbon dioxide external administration device 1, when the carbon dioxide absorption aid 4 is applied to the skin or mucosa, it is preferable to form a film of the carbon dioxide-dissolving medium on the skin or mucosa as thin as possible. When the film of the carbon dioxide-dissolving medium is too thick, it takes time for the carbon dioxide to dissolve in the medium, and to diffuse and be absorbed into the skin or mucosa, and hence the carbon dioxide absorption efficiency may be poor.

However, in the case that the vaporization temperature of the carbon dioxide-dissolving medium is relatively low, when the film of the carbon dioxide-dissolving medium formed is too thin, the medium may vaporize or evaporate due to the skin temperature and hence may be lost from the skin or mucosa while the carbon dioxide is being absorbed; the amount of the carbon dioxide-dissolving medium to be used must thus be suitably adjusted.

Raw materials commonly used in external preparations and cosmetics, for example fragrances, colorants, surfactants, oils, moisturizers, alcohols, preservatives, antioxidants, sequestering agents, anti-colorants, ultraviolet absorbing/scattering agents, vitamins, amino acids, drugs such as arbutin, kojic acid, nutrients, anti-inflammatories, vasodilators, hormones, astringents, antihistamines, microbicides, sebum inhibiting agents, keratin stripping/dissolving agents, anti-seborrheic agents and antipruritics, and so on, may be blended into the carbon dioxide absorption aid 4, whereby the carbon dioxide absorption aid 4 can be used yet more suitably for a cosmetic or medical purpose.

With the carbon dioxide external administration device 1 used in the present invention, the carbon dioxide used is gaseous, and the proportion of carbon dioxide in the gas is preferably not less than 10 %, more preferably not less than 30 %. There is no upper limit in the ratio. Administration amount of carbon dioxide is preferably 0.1 mg/cm² of the skin mucosa or more, preferably 0.3 mg/cm² of the skin mucosa or more.

### B. Use of the carbon dioxide external administration device (5):

Next, embodiments of the present invention is explained below when the carbon dioxide external administration device (5) was used with reference to the drawings. In addition, the same sign means the same element or corresponding element in each drawing.

A carbon dioxide external administration device 1 according to the present invention includes at least: a sealing enclosure member 2 capable of sealing off a body surface from outside air; a supply unit 3 for supplying carbon dioxide to the inside of the sealing enclosure member 2; and a perspiration promoting means 4 for promoting perspiration at the body surface inside the sealing enclosure member 2.

It is to be noted that the sealing enclosure member 2 does not have to completely seal off the body surface from outside air, but it may be only necessary to continually supply a small amount of carbon dioxide to the sealing enclosure member 2, thereby maintaining the concentration of carbon dioxide inside the sealing enclosure member 2 at or above a determined level.

### FIRST EMBODIMENT

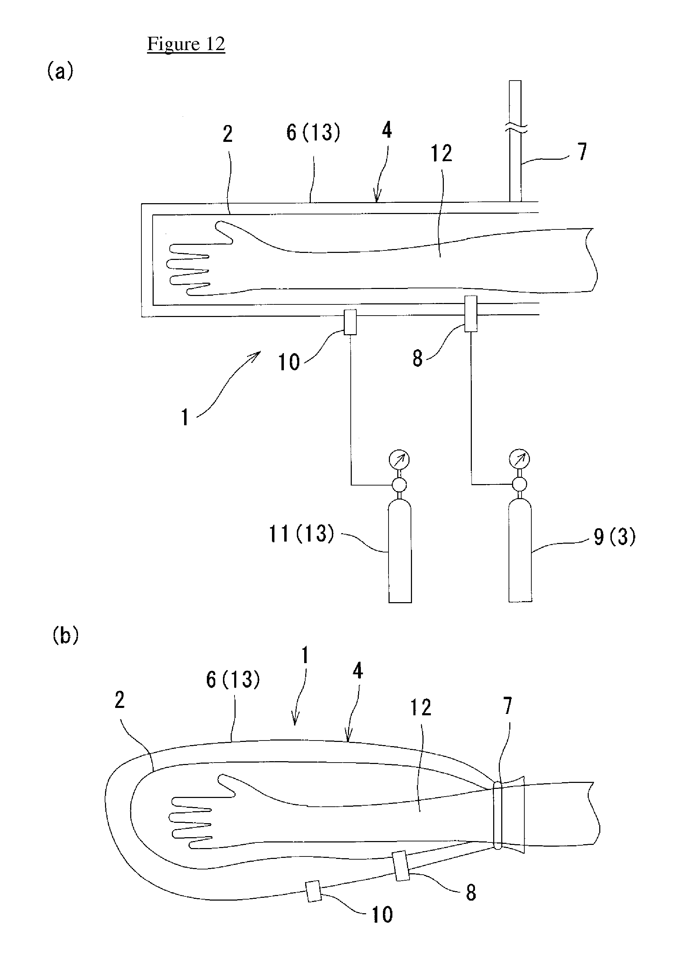

FIG. 12 shows the carbon dioxide external administration device 1 according to a first embodiment of the present invention.

As shown in FIG. 12(a), in the administration device 1 of the present embodiment, the sealing enclosure member 2 is a bag body opened at its one end side, and made of flexible synthetic resin. The sealing enclosure member 2 can cover a body part from a fingertip of a hand to an arm. This bag body is surrounded at its periphery by a pressurizing enclosure member 6, thereby providing a dual inside and outside structure. The pressurizing enclosure member 6 is also a bag body opened at its one end side, and made of synthetic resin. The sealing enclosure member 2 is provided at its opening with a tightening band 7 that is bound around an arm 12, thereby securing the airtightness of the inside of both the enclosure members 2 and 6.

A first coupler 8 having a check valve function is attached to the sealing enclosure member 2. A discharge port of the first coupler 8 airtightly passes through the sealing enclosure member 2 so as to be communicated with the inside of the sealing enclosure member 2. A connection port of the first coupler 8 is protruded to the outside of the pressurizing enclosure member 6. Hence, a hose of a first tank 9 for storing carbon dioxide is connected to the connection port of the first coupler 8, and carbon dioxide is discharged from the first tank 9, thereby enabling the supply of carbon dioxide to the inside of the sealing enclosure member 2. Accordingly, in the present embodiment, the first tank 9 constitutes the carbon dioxide supply unit 3.

A second coupler 10 having a check valve function is attached to the pressurizing enclosure member 6. A discharge port of the second coupler 10 airtightly passes through the pressurizing enclosure member 6 so as to be communicated with the inside of the pressurizing enclosure member 6. A connection port of the second coupler 10 is protruded to the outside of the pressurizing enclosure member 6.

Hence, a hose of a second tank 11 for storing air is connected to the connection port of the second coupler 10, and air is discharged from the second tank 11, thereby enabling the supply of air to a space formed between the pressurizing enclosure member 6 and the sealing enclosure member 2. Accordingly, in the present embodiment, the second tank 11 constitutes an air supply unit.

When the administration device 1 of the present embodiment is used, as shown in FIG. 12(a), the arm 12 of a treated person is inserted from his or her fingertip through the opening of the sealing enclosure member 2, and the tightening band 7 is bound around the arm 12, thereby airtightly closing the openings of the sealing enclosure member 2 and the pressurizing enclosure member 6.

In this state, carbon dioxide is supplied from the first tank 9 to the inside of the sealing enclosure member 2, and at the same time, air is supplied from the second tank 11 to the space formed between the sealing enclosure member 2 and the pressurizing enclosure member 6.

As a result, as shown in FIG. 12(b), the inside of the sealing enclosure member 2 is almost filled with carbon dioxide, and the sealing enclosure member 2 is pressurized from outside by the air pressure of the inside of the pressurizing enclosure member 6, thus pressurizing gas inside the sealing enclosure member 2 by a pressure exceeding atmospheric pressure.

Therefore, in the present embodiment, the pressurizing enclosure member 6 and the second tank 11 for supplying air to the inside of the pressurizing enclosure member 6 constitute a pressurizing member 13 for pressurizing the gas inside the sealing enclosure member 2 to a pressure exceeding atmospheric pressure.

As apparent from examples described above, if the pressure of the carbon dioxide-containing gas, which is brought into contact with the body surface, is pressurized to a pressure exceeding atmospheric pressure as described above, a sensation of warmth of the entire arm 12 serving as a region to be treated is considerably intensified, and a sensation of warmth is also caused in a region other than the region to be treated, thereby causing perspiration at the arm 12 and the other region of the treated person. Accordingly, the pressurizing member 13 of the present embodiment functions as the perspiration promoting means 4 for promoting perspiration at the body surface inside the sealing enclosure member 2.

It should be noted that the pressure of carbon dioxide inside the sealing enclosure member 2 for causing perspiration at the body surface of the treated person is approximately at a pressure of about 1.1 to about 5 atmospheres. This is because in the case of a pressure of less than 1.1 atmospheres, no pressurizing effect can be obtained, and in the case of a pressure of above 5 atmospheres, further medical effects or cosmetic effects cannon be obtained.

Carbon dioxide dissolves in the sweat of the body surface, caused by the perspiration of the treated person himself or herself, and this dissolved carbon dioxide is effectively absorbed percutaneously and transmucosally. Hence, since the sweat consequently serves as an absorption aid for carbon dioxide, strong medical effects, cosmetic effects, and fatigue recovery effects can be obtained in a short period of time.

Accordingly, the administration device 1 of the present embodiment is capable of obtaining medical effects, cosmetic effects, effects of recovery from fatigue, etc. in a short period of time regardless of whether or not an absorption aid 25 described later is provided in advance inside the sealing enclosure member 2.

Further, in the present embodiment, the pressurizing enclosure member 6 surrounds the periphery of the sealing enclosure member 2, and air is supplied to the space between both of the enclosure members 2 and 6; therefore, the gas inside the sealing enclosure member 2 can be pressurized even if the supply amount of carbon dioxide is not increased. Hence, as compared with the case where pressurization is carried out by increasing the supply amount of carbon dioxide, the inside of the sealing enclosure member 2 can be more inexpensively pressurized, and treatment by percutaneous and transmucosal absorption of carbon dioxide can be more inexpensively carried out.

### SECOND EMBODIMENT

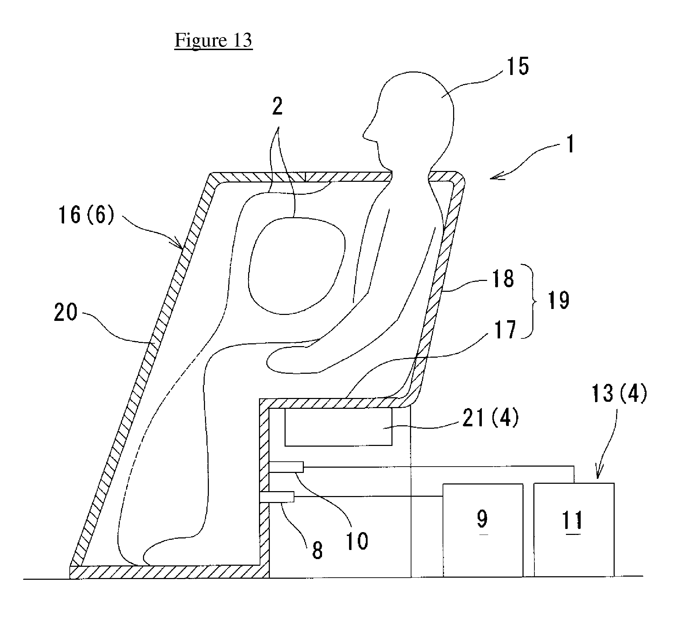

FIG. 13 shows a carbon dioxide external administration device 1 according to a second embodiment of the present invention.

As shown in FIG. 13, in the administration device 1 of the present embodiment, the pressurizing enclosure member 6 is a hard and hollow box body 16, and is capable of accommodating the body of a treated person 15 entirely below his or her neck. The box body 16 includes: a main body part 19 having a seat part 17 and a backrest part 18; and a front lid part 20 for sealing off a front opening of the main body part 19 in an openable and closable manner.

The box body 16 is provided at its inner face side with the sealing enclosure member 2. The sealing enclosure member 2 is a bag body made of flexible synthetic resin, and is capable of accommodating the body of the treated person 15 entirely below his or her neck.

The first coupler 8 and the second coupler 10, each having a check valve function, are attached to a lower part of the box body 16. Further, from the first tank 9 connected to the first coupler 8, carbon dioxide can be supplied to the inside of the sealing enclosure member 2. Furthermore, from the second tank 11 connected to the second coupler 10, air can be supplied to a space formed between the sealing enclosure member 2 and the pressurizing enclosure member 6.

In the present embodiment, at a lower face of the seat part 17 of the box body 16, a heating member 21 including a heating wire heater or the like is provided. The heating member 21 can heat the inside of the sealing enclosure member 2 to a temperature exceeding the skin temperature. It should be noted that the skin temperature of the treated person is at a low temperature (about 25 °C) which is generally lower than a body temperature (about 36 °C), and therefore, it is only necessary for the heating member 21 to be capable of heating the inside of the sealing enclosure member 2 to a temperature exceeding the above-mentioned low temperature.

When the administration device 1 of the present embodiment is used, as shown in FIG. 13, the treated person 15 is allowed to sit on the seat part 17 of the box body 16 with only his or her neck exposed to the outside, and the front lid part 20 is closed to airtightly close the box body 16.

In this state, carbon dioxide is supplied from the first tank 9 to the inside of the sealing enclosure member 2, and at the same time, air is supplied from the second tank 11 to the space formed between the sealing enclosure member 2 and the pressurizing enclosure member 6.

As a result, the inside of the sealing enclosure member 2 is almost filled with carbon dioxide, and the sealing enclosure member 2 is pressurized from outside by the air pressure of the inside of the pressurizing enclosure member 6, thus pressurizing the gas inside the sealing enclosure member 2 by a pressure exceeding atmospheric pressure. Therefore, also in the present embodiment, the pressurizing enclosure member 6 and the second tank 11 for supplying air to the inside of the pressurizing enclosure member 6 constitute the pressurizing member 13 for pressurizing the gas inside the sealing enclosure member 2 to a pressure exceeding atmospheric pressure.

As apparent from the examples described later, if the pressure of the carbon dioxide-containing gas, which is brought into contact with the body surface, is pressurized to a pressure exceeding atmospheric pressure as described above, a sensation of warmth of the entire body below the neck, serving as a region to be treated, is considerably intensified, thereby causing perspiration of the treated person 15. Accordingly, the pressurizing member 13 of the present embodiment also functions as the perspiration promoting means 4 for promoting perspiration at the body surface inside the sealing enclosure member 2.

Furthermore, in the second embodiment, the inside of sealing enclosure member 2 is heated to a temperature exceeding the skin temperature by the heating member 21 provided at the seat part 17, thus facilitating the perspiration of the treated person 15. Therefore, in the present embodiment, the heating member 21 also functions as the perspiration promoting means 4 for promoting perspiration at the body surface.

Carbon dioxide dissolves in the sweat of the body surface, caused by the perspiration of the treated person 15 himself or herself, and this dissolved carbon dioxide is effectively absorbed percutaneously and transmucosally. That is, since the sweat consequently serves as an absorption aid for carbon dioxide, strong medical effects, cosmetic effects, and fatigue recovery effects can be obtained in a short period of time.

As described above, the administration device 1 of the present embodiment is also capable of obtaining medical effects, cosmetic effects, effects of recovery from fatigue, etc. in a short period of time regardless of whether or not the absorption aid 25 described later is provided in advance inside the sealing enclosure member 2.

Further, in the present embodiment, the pressurizing enclosure member 6 surrounds the periphery of the sealing enclosure member 2, and air is supplied to the space between both of the enclosure members 2 and 6; therefore, the gas inside the sealing enclosure member 2 can be pressurized even if the supply amount of carbon dioxide is not increased. Therefore, as compared with the case where pressurization is carried out by increasing the supply amount of carbon dioxide, the inside of the sealing enclosure member 2 can be more inexpensively pressurized, and treatment by percutaneous and transmucosal absorption of carbon dioxide can be more inexpensively carried out.

### OTHER EMBODIMENTS

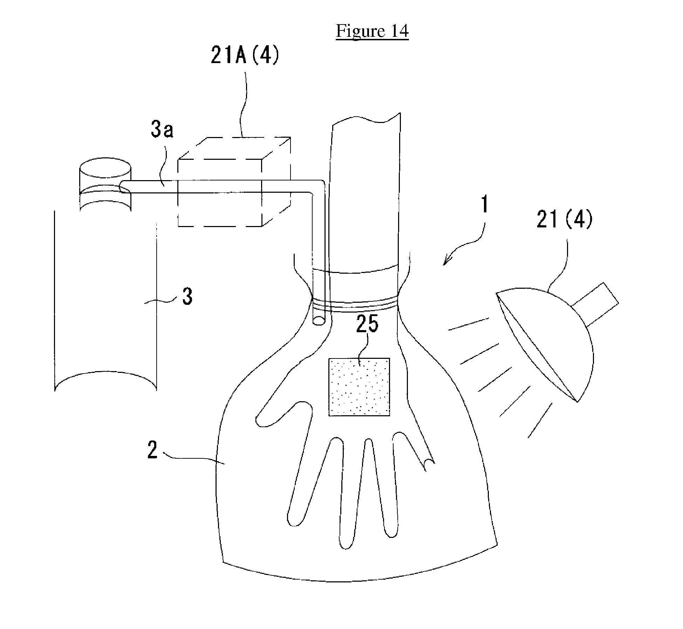

FIGs. 14 to 17 show third to sixth embodiments of the present invention, respectively.

As shown in FIGs. 14 to 17, in the administration device 1 of the present invention, the shape and material of the sealing enclosure member 2 are not particularly limited as long as the member can cover the skin or mucosa and maintain a certain space in which carbon dioxide is held.

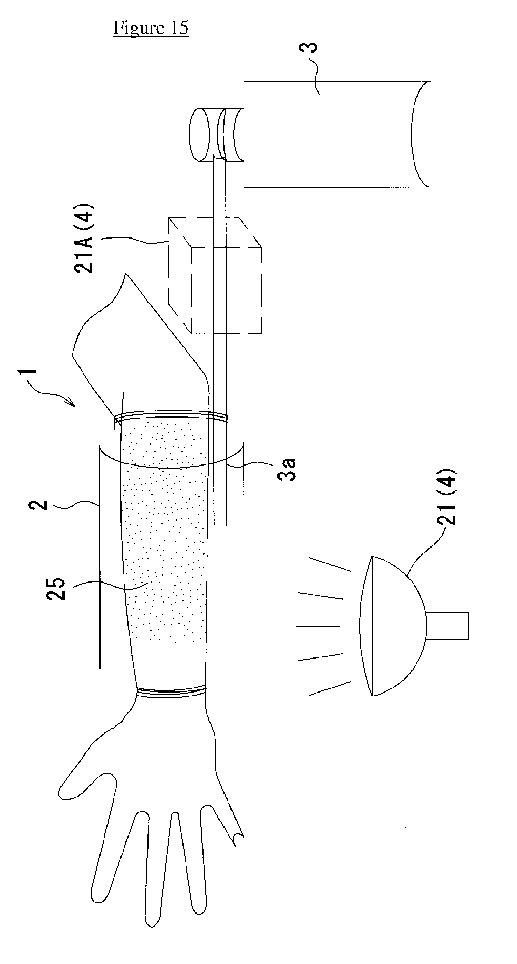

As the shape of the sealing enclosure member 2, it is possible to adopt a bag body capable of covering a hand (or a foot) as shown in FIG. 14, or a tubular bag body that can be wound around an arm (or a foot) as shown in FIG. 15, for example.

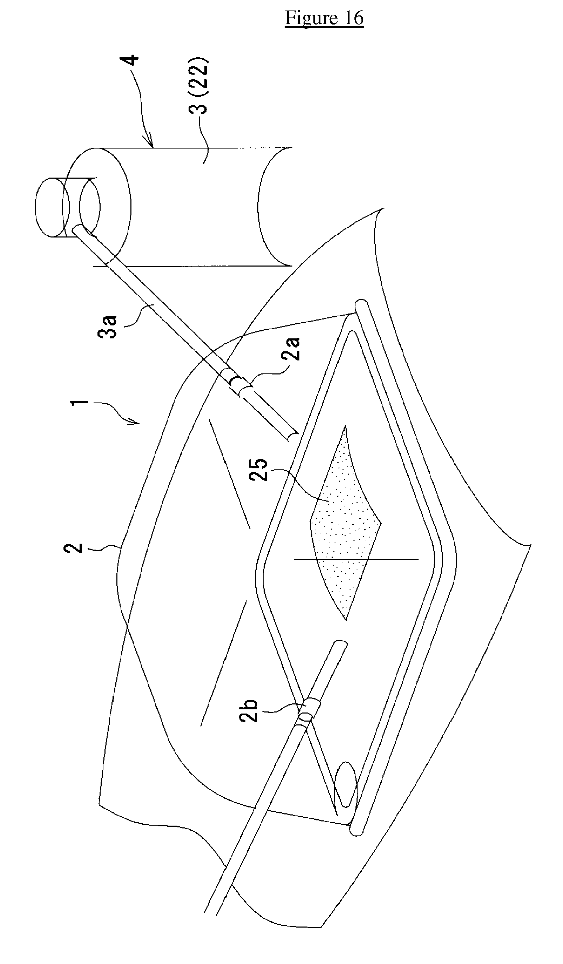

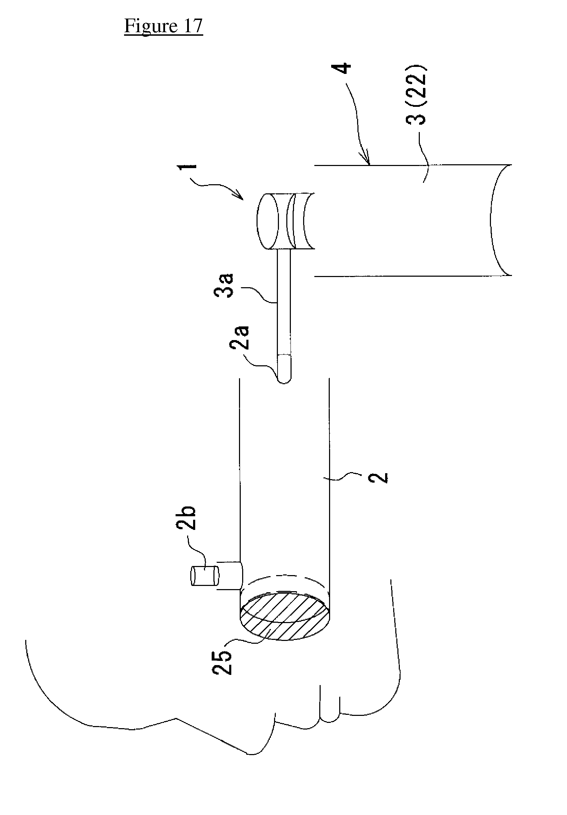

Further, as the shape of the sealing enclosure member 2, it is possible to adopt a container having an opened lower edge that is brought into tight contact with a skin surface with a relatively wide area such as abdomen as shown in FIG. 16, or a cup-shaped cylindrical body, one end of which is opened and the other end of which serves as a connection with the supply unit 3 as shown in FIG. 17. Naturally, the shape of the sealing enclosure member 2 is not limited to these examples.

In the third embodiment of FIG. 14 and the fourth embodiment of FIG. 15, the heating member 21 including a far infrared heater or the like is provided as a constituent element of the administration device 1. The heating member 21 is capable of heating the inside of the sealing enclosure member 2 to a temperature exceeding the skin temperature, and functions as the perspiration promoting means 4 for promoting perspiration of the treated person.

As indicated by virtual lines in FIGs. 14 and 15, a tube 3a, through which carbon dioxide is supplied, may be allowed to pass through a heating member 21A including a thermoregulated water bath or the like. In this case, when carbon dioxide is supplied from the carbon dioxide supply unit (tank) 3, carbon dioxide heated by the heating member 21 A is supplied to the inside of the sealing enclosure member 2, thereby promoting the perspiration of the treated person. Accordingly, the heating member 21A constitutes the perspiration promoting means 4.

In the fifth embodiment of FIG. 16 and the sixth embodiment of FIG. 17, together with carbon dioxide, steam is enclosed in the inside of the carbon dioxide supply unit (tank) 3 serving as a constituent element of the administration device 1. Furthermore, the supply unit 3 can simultaneously supply carbon dioxide and steam to the inside of the sealing enclosure member 2, and can humidify the inside of the sealing enclosure member 2.

Therefore, in the fifth and sixth embodiments, the carbon dioxide supply unit 3 also functions as a humidifying member 22 for increasing a humidity of the inside of the sealing enclosure member 2 to a humidity exceeding the room humidity. Naturally, in addition to the carbon dioxide supply unit 3, a humidifier (humidifying member) may be connected to the sealing enclosure member 2, thereby separately supplying steam by this humidifier.

Upon increase of the humidity of the inside of the sealing enclosure member 2, perspiration through the body surface is promoted, and therefore, the humidifying member 22 functions as the perspiration promoting means 4 for promoting the perspiration of the treated person.

Next, the respective constituent elements of the present invention will be described.

### SEALING ENCLOSURE MEMBER

As described in each of the foregoing embodiments, the constituent material of the sealing enclosure member 2 is not particularly limited as long as it is a gas impermeable material. For example, a material such as metal, plastic, rubber, or glass may be appropriately selected and used in accordance with an objective or an applying region.

If the sealing enclosure member 2 is formed by a non-elastic hard material such as metal or glass (the sealing enclosure member in this case will hereinafter be called a "robust type sealing enclosure member"), the sealing enclosure member 2 preferably has a shape that surrounds an applying region so as to maintain a certain sealed-off space. The skin or mucosa contacting portion preferably has a shape conforming to the applying region so as to prevent the leakage of carbon dioxide.

However, an elastic material such as rubber or resin can flexibly conform to the applying region, and is therefore preferably used in combination with the skin or mucosa contacting region. For example, a material such as a viscoelastic gel is more preferably used because it has a higher flexibility and an excellent adherence to the skin or mucosa.

For example, the tube 3a extending from the carbon dioxide supply unit 3 is connected to a gas injection port 2a (see FIGs. 16 and 17) of the robust type sealing enclosure member 2. The gas injection port 2a is not particularly limited as long as it has a structure that prevents leakage of gas, but more preferably includes a check valve for preventing backflow of gas. A gas discharge port 2b (see Figs. 16 and 17) of the robust type sealing enclosure member 2 may be a gap in the skin or mucosa contacting region, but more preferably includes a check valve for preventing backflow of gas in order to reliably and efficiently carry out the replacement of air inside the sealing enclosure member 2 with carbon dioxide.

If the sealing enclosure member 2 is formed by a material capable of maintaining its form, i.e., a flexible material such as rubber, resin or soft plastic, which is not significantly deformed even if some external force is applied thereto (the sealing enclosure member in this case will hereinafter be called a "flexible type sealing enclosure member"), the skin or mucosa contacting region of the sealing enclosure member 2 is also soft, and can therefore be used as it is.

However, since a material such as a viscoelastic gel has a higher flexibility and an excellent adherence to the skin or mucosa, such a material is more preferably used in combination with the skin or mucosa contacting region. It should be noted that also in the flexible type sealing enclosure member 2, the gas injection port and gas discharge port similar to those of the robust type sealing enclosure member 2 can be adopted.

If the sealing enclosure member 2 is formed by a flexible material such as rubber or resin, which has a high elasticity like a balloon (the sealing enclosure member in this case will hereinafter be called an "elastic sealing enclosure member"), the sealing enclosure member 2 can be used in the form of a cylindrical shape or a bag-like shape, for example. In this case, an opening of the cylinder, bag or the like covering a desired region is bound, or an elastic open/close port, to which rubber, spring or the like is attached to its opening, is provided, thus enabling the sealing off of the skin or mucosa.

Further, in the case of the cylindrical elastic sealing enclosure member 2, the applying region of which is an arm, for example, the diameter of the periphery of the cylinder may be set to be substantially similar to or less than that of the periphery of the arm. In this case, the sealing enclosure member 2 may seal off the skin or mucosa in a state where the sealing enclosure member 2 is adhered to the skin or mucosa or the space between the sealing enclosure member 2 and the skin or mucosa is extremely small, and the sealing enclosure member 2 may be expanded by injection of carbon dioxide into the space between the sealing enclosure member 2 and the skin or mucosa, thereby forming a certain space, in which carbon dioxide is held, by the injected carbon dioxide itself.

The gas injection port and gas discharge port of the elastic sealing enclosure member 2 may have the same structures as in the case of the robust type sealing enclosure member 2. However, in the elastic sealing enclosure member 2 in the form of a cylindrical shape, a bag-like shape or the like, the skin or mucosa may be sealed off by the cylinder, bag or the like, inside air may be discharged in advance as much as possible, the tube 3a may be inserted into an opening of the cylinder, bag or the like while air is prevented, to the extent possible, from going inside, and then carbon dioxide may be injected (see FIGs. 14 and 15).

If the sealing enclosure member 2 is formed by a foldable sheet-like or film-like material (the sealing enclosure member in this case will hereinafter be called a "sheet type sealing enclosure member"), the sealing enclosure member 2 can be used in the form of a cylindrical shape, a bag-like shape or the like. In this case, an opening of the cylinder, bag or the like covering a desired region is bound, or an elastic open/close port, to which rubber, spring or the like is attached to its opening, is provided, thus enabling the sealing off of the skin or mucosa.

Further, in the case of the cylindrical sheet type sealing enclosure member 2, the applying region of which is an arm (or a foot), for example, the diameter of the periphery of the cylinder may be set to be greater than that of the periphery of the arm. In this case, the sealing enclosure member 2 may seal off the arm, the sealing enclosure member 2 itself may be folded, inside air may be discharged as much as possible, and then the sealing enclosure member 2 may be used while being adhered to the skin or mucosa.

The gas injection port and gas discharge port of the sheet type sealing enclosure member 2 may have the same structures as in the case of the robust type sealing enclosure member 2. However, in the sheet type sealing enclosure member 2 in the form of a cylindrical shape, a bag-like shape or the like, the skin or mucosa may be sealed off by the cylinder, bag or the like, inside air may be discharged in advance as much as possible, the tube may be inserted into an opening of the cylinder, bag or the like while air is prevented, to the extent possible, from going inside, and then carbon dioxide may be injected.

### CARBON DIOXIDE SUPPLY UNIT

In the administration device 1 of the present invention, the carbon dioxide supply unit 3 is not particularly limited, and a commercially available carbon dioxide gas tank or the like, for example, can be used, or a sealed-off container provided with a tube, for example, can be used as follows. Specifically, carbon dioxide is generated by vaporizing dry ice, which is solid carbon dioxide, inside the sealed-off container, or by a reaction between carbonate and acid inside the sealed-off container.

In the administration device 1 of the present invention, it is only necessary for the tube 3a for connecting the sealing enclosure member 2 with the supply unit 3 to prevent leakage of gas to the outside. As the tube 3a, any material such as rubber, resin, metal or glass may be used with no particular limitation as long as it can be molded into a tube.

### CARBON DIOXIDE

In the administration device 1 of the present invention, carbon dioxide to be used is in gaseous state, and the proportion of carbon dioxide in this gas is preferably 10 % or more, and more preferably 30 % or more. A dose of carbon dioxide is preferably 0.1 mg or more, and more preferably 0.3 mg or more per square centimeter of the skin or mucosa.

### ABSORPTION AID

As shown in each of the embodiments of FIGs. 14 to 17, in the administration device 1 of the present invention, in addition to the adoption of the perspiration promoting means 4, the carbon dioxide absorption aid 25 may additionally be adopted. Examples of a material used for the absorption aid 25 are as follows, and include: a sheet-like material; a viscous material; alcohols; fats and oils; and waxes.

The above-mentioned sheet-like material is not particularly limited as long as the material can be impregnated with a liquid containing at least water and can be affixed to the skin or mucosa. Examples of the above-mentioned sheet-like material include: a woven fabric or a nonwoven fabric consisting of natural fiber, synthetic fiber and/or semi-synthetic fiber; a semi-permeable membrane such as a cellulose membrane; and a hydrogel sheet consisting of naturally-occurring polymers, synthetic polymers and/or semi-synthetic polymers, and one or more of these can be used.

The above-mentioned liquid containing at least water is not particularly limited, and the liquid may be water itself or may be water in which some substance is dissolved or dispersed as long as the effects of the present invention are not impaired. Further, the above-mentioned liquid containing at least water preferably has a pH value of 7 to 2, or the liquid is more preferably acidic water having a pH value of 6.5 to 4. This is because carbon dioxide is efficiently absorbed percutaneously and transmucosally when dissolved in an acidic solvent having a pH value of 4 or more.

The above-mentioned viscous material may be in liquid form or in semi-solid form as long as the material contains water, does not easily flow down when applied to the skin or mucosa, and has a viscosity of 20 cps or more at 20 °C. Examples of a preparation for the above-mentioned viscous material include: liquid; cream; paste; and gel, and one or more of these can be used.

The above-mentioned viscous material preferably has a pH value of 7 to 2, and is more preferably an acidic viscous material having a pH value of 6.5 to 4.

The above-mentioned viscous material is not particularly limited, but is preferably a material having moisture content as high as possible, and capable of replenishing the skin or mucosa with moisture. As a liquid serving as the preparation, a thickener solution, suspension, or swelling liquid, for example, can be suitably used because a necessary viscosity can be obtained with a small amount, or adherence or adhesion to the skin or mucosa is excellent. Methods for producing these liquids are not particularly limited, and public known methods can be adopted. Cream, paste or gel serving as the preparation is not particularly limited, and can be used by producing it by a public known method.

As the above-mentioned thickener used for the viscous material, one or more thickeners selected from a group consisting of: natural polymers; semi-synthetic polymers; synthetic polymers; and inorganic substances can be used.

Examples of the natural polymers include: plant-derived polymers such as gum arabic, carrageenan, galactan, agar, xanthan gum, quince seed gum, guar gum, tragacanth, pectin, manna, locust bean gum, wheat starch, rice starch, corn starch and potato starch; microorganism- derived polymers such as curdlan, xanthan gum, succinoglucan, dextran, hyaluronic acid and pullulan; and protein-type polymers such as albumin, casein, collagen, gelatin and fibroin, and one or more of these can be used.

Among the above-mentioned natural polymers, gum arabic, carrageenan, xanthan gum, tragacanth, hyaluronic acid, pullulan, pectin, mannan, and locust bean gum are preferable in terms of affinity for the skin or mucosa and the like, and furthermore, carrageenan and pectin are more preferable in terms of sense of use and the like.

Examples of the semi-synthetic polymers include: cellulosic polymers such as ethylcellulose, carboxymethyl cellulose, carboxymethylethylcellulose, carboxymethyl starch, croscarmellose, crystalline cellulose, cellulose acetate, cellulose acetate phthalate, hydroxyethylcellulose, hydroxypropylcellulose, hydroxypropylmethylcellulose, hydroxypropylmethylcellulose phthalate, methylcellulose and methylhydroxypropylcellulose; starch-type polymers such as pregelatinized starch, partially pregelatinized starch, carboxymethyl starch, dextrin, methyl starch, starch-acrylic acid copolymers and cellulose-acrylonitrile graft copolymers; alginate-type polymers such as sodium alginate and propylene glycol alginate; and other polysaccharide-type polymers such as sodium chondroitin sulfate and sodium hyaluronate, and one or more of these can be used.

Among the above-mentioned semi-synthetic polymers, sodium alginate, propylene glycol alginate, carboxymethylcellulose sodium, dextrin, and sodium hyaluronate are preferable in terms of affinity for the skin or mucosa and the like, and furthermore, sodium alginate, propylene glycol alginate, and sodium hyaluronate are more preferable in terms of sense of use and the like.

Examples of the synthetic polymers include: carboxyvinyl polymer; sodium polyacrylate; polyamine; polyacrylamide; polyvinylacetal diethylaminoacetate; polyvinyl alcohol; polyvinylpyrrolidone; polyethylene glycol; polyethylene oxide; poly(meth)acrylic acid; and polyvinyl methyl ether, and one or more of these can be used.

Among the above-mentioned synthetic polymers, carboxyvinyl polymer, sodium polyacrylate, polyacrylamide, polyvinyl alcohol, and polyvinylpyrrolidone are preferable in terms of affinity for the skin and mucosa and the like, and furthermore, polyvinyl alcohol, polyvinylpyrrolidone, and carboxyvinyl polymer are more preferable in terms of sense of use and the like.

Examples of the inorganic substances include: hydrated silicon dioxide; light anhydrous silicic acid; colloidal alumina; bentonite; and laponite, and one or more of these can be used.

The above-mentioned alcohols are not particularly limited as long as they are in liquid form or in semi-solid form at normal temperature, and are not easily vaporized at human skin temperature. Examples of the above-mentioned alcohols include: monohydric alcohols such as isopropyl alcohol and 1-butanol; and polyhydric alcohols such as ethylene glycol, diethylene glycol, polyethylene glycol, propylene glycol, dipropylene glycol, 1,3-butanediol, 1,2-pentanediol, isoprene glycol, glycerol, diglycerol, triglycerol and tetraglycerol, and one or more of these can be used.

A method for using the alcohols vaporized at high temperature is not particularly limited. The above-mentioned alcohols may be used in accordance with physical properties, etc. thereof by being directly applied or sprayed onto the skin or mucosa, or by being impregnated into a nonwoven fabric or the like so as to be affixed to the skin or mucosa.

The above-mentioned fats and oils are not particularly limited as long as they are in liquid form or in semi-solid form at normal temperature, and can be relatively thinly applied to the skin or mucosa. Examples of the above-mentioned fats and oils include: natural vegetable fats and oils such as avocado oil, avocado butter, olive oil, sesame oil, safflower oil, soybean oil, camellia oil, sunflower oil and macadamia nut oil; and animal fats and oils such as squalane, mink oil, beef fat, lard, chicken fat and horse fat, and one or more of these can be used.

A method for using the above-mentioned fats and oils is not particularly limited. The above-mentioned fats and oils may be used in accordance with physical properties, etc. thereof by being directly applied or sprayed onto the skin or mucosa, or by being impregnated into a nonwoven fabric or the like so as to be affixed to the skin or mucosa.

The above-mentioned waxes are not particularly limited as long as they are in liquid form or in semi-solid form at normal temperature, and can be relatively thinly applied to the skin or mucosa. Examples of the above-mentioned waxes include: jojoba oil; carnauba wax; candelilla wax; beeswax; and lanolin, and one or more of these can be used.

A method for using the above-mentioned waxes is not particularly limited. The above-mentioned waxes may be used in accordance with physical properties, etc. thereof by being directly applied or sprayed onto the skin or mucosa, or by being impregnated into a nonwoven fabric or the like so as to be affixed to the skin or mucosa.

When the absorption aid 25 is applied to the skin or mucosa in the case of the administration device 1 of the present invention, it is preferable that a film of a carbon dioxide-dissolving medium is formed on the skin or mucosa as thin as possible. If the film of the carbon dioxide-dissolving medium is too thick, it takes time for carbon dioxide to be dissolved in the medium, and to be diffused and absorbed into the skin or mucosa, and the absorption efficiency of carbon dioxide might be degraded.

However, if the film of the carbon dioxide-dissolving medium to be formed is too thin when the vaporization temperature of the carbon dioxide-dissolving medium is relatively low, the medium might be vaporized or evaporated and lost from the skin or mucosa due to the skin temperature during absorption of carbon dioxide; therefore, the amount of the carbon dioxide-dissolving medium to be used must be adjusted.

Raw materials commonly used for external preparations or cosmetics can be mixed into the absorption aid 25, and such raw materials can be more suitably used in order to obtain medical effects, cosmetic effects or effects of recovery from fatigue. Examples of such raw materials include: a perfume; a colorant; a surfactant; an oil; a moisturizer; alcohols; a preservative; an antioxidant; a sequestering agent; an anti-coloring agent; an ultraviolet absorbing/scattering agent; vitamins; amino acids; arbutin; kojic acid; a nutrient; an anti-inflammatory agent; a vasodilator; a hormonal agent; an astringent; an antihistamine; a microbicide; a sebum inhibiting agent; a keratin removing/dissolving agent; an anti-seborrheic agent; and an antipruritic.

The administration device 1 of the present invention is applicable not only to humans but also to animals. Animals to which the administration device is applied are not particularly limited as long as they have sweat glands. Specifically, examples of such animals include horses and cattle. For animals having substantially few sweat glands, such as dogs and cats, percutaneous and transmucosal absorption of carbon dioxide can be promoted by using the administration device 1 of the present invention including one or both of: the heating member for heating the inside of the sealing enclosure member to a temperature exceeding the skin temperature; and the humidifying member for increasing the humidity of the inside of the sealing enclosure member to a humidity exceeding the room humidity. The pressurization of gas inside the sealing enclosure member to a pressure exceeding atmospheric pressure is also effective for animals having substantially few sweat glands.

Also for primates, poikilothermal animals and the like, the above-described method for animals having sweat glands or animals having substantially few sweat glands is employed as necessary, and the administration device 1 of the present invention is used, thus making it possible to easily obtain medical effects, cosmetic effects and the like resulting from carbon dioxide.

In a therapeutic agent for fracture, a bone growth promoting agent, or a therapeutic or prophylactic agent for bone diseases containing carbon dioxide as an active ingredient in the present invention, concentration and period for the carbon dioxide absorption depend on a method for carbon dioxide absorption, but the carbon dioxide concentration equal to or over 300 ppm is preferable, and that equal to or over 1000 ppm is more preferable.

There is no upper limit in the carbon dioxide concentration. Usually a period for carbon dioxide absorption equal to or over 5 minutes is preferable, and that equal to or over 10 minutes is more preferable. Usually 30 minutes of carbon dioxide absorption is enough for obtaining a desired effect, though there is no problem in absorbing carbon dioxide for long period since carbon dioxide is harmless.

As to frequency of carbon dioxide absorption, once a week is preferable and once or more a day is further preferable.

Usually the fracture part is immobilized in a plaster cast in fracture treatment and it is not easy to administrate a therapeutic agent for bone fracture comprising carbon dioxide as an active ingredient, but immobilization simultaneously with carbon dioxide absorption is possible, if the plaster cast is designed to have a function of the sealing enclosure member described in WO 2004/517345. Needless to say, carbon dioxide absorption is possible if the plaster cast is detached.

In human, bone growth in the direction of the long axis of the bone is terminated after the period of growth, so a bone growth promoting treatment should be done to a child in the period of growth in order to enhance growing taller. In general the period of growth means till 17 years old in a boy and till 14 years old in a girl. In an animal having continuous period of growth such as a rat for example, an effect on promoting bone growth is available without depending on the age when administered.

In case that a bone resorption inhibitor and/or an osteogenesis promoting agent is administered together with a therapeutic agent for bone fracture of the present invention comprising carbon dioxide as an active ingredient, carbon dioxide may be absorbed simultaneously with the administration of these agent, or independently on the administration.

Examples of the bone resorption inhibitors and/or the osteogenesis promoting agents mentioned above include activated vitamin D3, estrogen, estrogenic substance, calcitonin, ipriflavone, bisphosphonate, vitamin K, calcium, parathyroid hormone, and a derivative thereof, for example.

The carbon dioxide external preparation, which is one of the means for absorption of carbon dioxide into the body in the present invention, is explained below.

Examples of the medium to dissolve carbon dioxide contained in the carbon dioxide external preparation include water, alcohol, fat and wax, and one or more of these materials may be used. Examples of water, alcohol, oil and fat, and wax listed in the carbon dioxide external administration device can be used in the same manner.

A method for preparing the carbon dioxide external preparation is exemplified as follows:
1) A method for dissolving carbon dioxide in the medium by directly blowing carbon dioxide therein;
2) A composition for preparing external carbon dioxide agent, in which carbon dioxide is generated in a medium to dissolve carbon dioxide by reacting an acid and a carbonate salt in the medium; and
3) A composition for preparing external carbon dioxide agent, comprising microorganism to generate carbon dioxide through fermentation in a medium to dissolve carbon dioxide, and materials necessary for the fermentation,
is exemplified.

Carbon dioxide dissolved in a medium is easily dissipated in the air and difficult to preserve it. Also, there is a limitation in the amount of carbon dioxide dissolved in the medium. Therefore, it is preferable to use the composition for preparing external carbon dioxide agent, in which carbon dioxide is continuously generated by the reaction between an acid and a carbonate salt in the medium to dissolve carbon dioxide, and the external agent is prepared at use.

Examples of the composition for preparing external carbon dioxide agent, in which carbon dioxide is generated by the reaction between an acid and a carbonate salt in the medium to dissolve carbon dioxide, are shown below:
(a) a composition for preparing external carbon dioxide agent consisting of a solid containing an acid and liquid containing a carbonate salt, in which the granular material and the liquid are mixed at use,
(b) a composition for preparing external carbon dioxide agent consisting of a solid containing a carbonate salt and liquid containing an acid, in which the granular material and the liquid are mixed at use,
(c) a composition for preparing external carbon dioxide agent, in which water is added to a solid containing an acid and a carbonate salt and mixed at use, and
(d) a composition for preparing external carbon dioxide agent consisting of liquid containing an acid and liquid containing a carbonate salt, in which these liquids are mixed at use.

More specifically, the following compositions for preparing external carbon dioxide agent are exemplified;
(e) a composition for preparing a carbon dioxide external agent characterized by comprising a substance generating an acid after being hydrolyzed, a carbonate, a thickener, and water as essential components, and further a gelating agent gelated by calcium ion, and a water-insoluble or poorly soluble calcium salt (WO 2006/80398);
(f) a composition for preparing an external carbon dioxide agent comprising a granular material containing a water-soluble acid, a thickener and a water-soluble dispersant as the essential components wherein the said thickener is mixed with the water-soluble acid and the water-soluble dispersant; and a carbonate, water and a thickener as the essential components, which are to be mixed with the said granular material at use (WO 2002/80941),
(g) a composition for preparing carbon dioxide gel for external use characterized by comprising granular material (A) and viscous material (B) to be mixed with the granular material (A):
   (A) a granular material comprising a weak acid and a calcium ion trapping agent as essential components;
   (B) a viscous material comprising calcium carbonate, a gelling agent to be gelated with calcium ions and water as essential components (WO 2005/16290), and
(h) a material for formation of carbon dioxide external preparation characterized by comprising a base agent that comprises a polymeric three-dimensional network structure impregnated with a viscous material containing at least an acid and water, and is made to contact with skin at use, and a reactant that contains at least a carbonate, and is made to contact with the base agent at use so as to generate carbon dioxide, the carbon dioxide being dissolved in the viscous material substantially in a non-bubble state (WO 2004/4745).

The carbon dioxide external agent prepared from e) a composition for preparing a carbon dioxide external agent above (hereinafter referred to a composition for preparing a carbon dioxide external agent [1]) is explained in detail:
The composition for preparing a carbon dioxide external agent [1] used in the present invention comprises a substance generating an acid after being hydrolyzed, a carbonate, a thickener, and water as essential components.

The substance generating an acid after being hydrolyzed in the present invention is preferably one or more selected from lactone, a cyclic dimer of organic acid, and acid anhydride, and more preferably one or more selected from the crystalline forms of lactone, a cyclic dimer of organic acid, and acid anhydride. Specific examples include glucono-delta-lactone; pantolactone; D,L- or L-lactide (3,6-dimethyl-1,4-dioxane-2,5-dione); D,L- or L-glycolide; phthalic anhydride; maleic anhydride; and succinic anhydride.

One of these examples or a combination of two or more of these examples may be used. Maleic anhydride and succinic anhydride are preferably used in combination with other substances generating an acid after being hydrolyzed such as glucono-delta-lactone or D,L-lactide to control the generating rate of an acid and consequently to control the generating rate of carbon dioxide at user's option since maleic anhydride and succinic anhydride are easily hydrolyzed and quickly generate an acid.

Any carbonate which generates carbon dioxide by the reaction with an acid may be used as a carbonate, and examples of the carbonate include ammonium carbonate, ammonium bicarbonate, potassium carbonate, potassium bicarbonate, potassium sesquicarbonate, sodium carbonate, sodium bicarbonate, sodium sesquicarbonate, lithium carbonate, lithium bicarbonate, lithium sesquicarbonate, cesium carbonate, cesium bicarbonate, cesium sesquicarbonate, magnesium carbonate, magnesium bicarbonate, calcium bicarbonate, calcium carbonate, magnesium hydroxide carbonate, or barium carbonate. One or more of these carbonates can be used and a water-soluble carbonate such as sodium bicarbonate or sodium carbonate is preferable.

There are no particular limitations on thickeners used and one or more selected from natural polymers, semi-synthetic polymers, synthetic polymers, and inorganic materials can be used.

Among them, examples of neutral or alkaline thickeners include the followings.

Examples of the natural polymers above include plant-originated polymers such as gum arabic, carrageenan, galactan, agar, quince seed, guar gum, tragacanth gum, mannan, locust bean gum, wheat starch, rice starch, tara gum, corn starch, and potato starch; microorganism-originated polymers such as curdlan, xanthan gum, succinoglucan, dextran, and pullulan; and protein polymers such as albumin, casein, collagen, gelatin and fibroin; one or more of these polymers can be used.

Examples of the semi-synthetic polymers above include cellulose polymers such as ethylcellulose, carboxymethylcellulose and its salts, carboxymethylethylcellulose and its salts, carboxymethyl starch and its salts, croscarmellose and its salts, crystalline cellulose, cellulose acetate, cellulose acetate phthalate, hydroxyethylcellulose, hydroxypropylcellulose, hydroxypropylmethylcellulose, hydroxypropylmethylcellulose phthalate, methylcellulose, and methylhydroxypropylcellulose; starch polymers such as pregelatinaized starch, partially pregelatinaized starch, carboxymethyl starch, dextrin, and methyl starch; alginate polymers such as sodium alginate, potassium alginate, ammonium alginate and propylene glycol alginate; and other polysaccharide polymers such as sodium chondroitin sulfate and sodium hyaluronate; and one or more of these polymers can be used.

Examples of the synthetic polymers above include sodium polyacrylate, polyvinylacetaldiethylaminoacetate, polyvinyl alcohol, polyvinyl pyrrolidone, methacrylic acid-ethyl acrylate copolymer, methacrylic acid - ethyl methacrylate copolymer, ethyl methacrylate - trimethylammoniumethyl chloride methacrylate copolymer, dimethylaminoethyl methacrylate-methyl methacrylate copolymer and the like. One or more of these polymers can be used.

Examples of the inorganic materials above include hydrated silicon dioxide, colloidal alumina, bentonite, laponite and the like; and one or more of these polymers can be used.

Examples of acidic thickeners include the followings.

Alginic acid, pectin and hyaluronic acid are included as a natural polymer; carboxyvinyl polymer is included as a semi-synthetic polymer and light anhydrous silicic acid is included as an inorganic material; one or more of these polymers or a material can be used.

There are no particular limitations on water used and natural water, tap water, distilled water, purified water and the like can be used.

A source of carbon dioxide in the composition for preparing a carbon dioxide external agent [1] is the substance generating an acid after being hydrolyzed and the carbonate. The substance generating an acid after being hydrolyzed is gradually hydrolyzed to generate an acid when contacted with water, and the acid reacts with the carbonate to generate carbon dioxide gradually. Next, the viscous material is formed by the thickener and the water included in the composition for preparing a carbon dioxide external agent [1] as essential components.

Since the rate of hydrolysis of the substance generating an acid after being hydrolyzed and of generating carbon dioxide subsequent to the hydrolysis is slower in the viscous material than in water, carbon dioxide is generated gradually and constantly, and it provides the carbon dioxide external agent in which the generated carbon dioxide is difficult to leak into the air.

In the composition for preparing a carbon dioxide external agent [1], the composition for preparing a carbon dioxide external agent is preferable, wherein a granular material is formed by components other than water (a substance generating an acid after being hydrolyzed, a carbonate and a thickener) (hereinafter, the composition for preparing a carbon dioxide external agent [1-1]), or one or more component(s) selected from a substance generating an acid after being hydrolyzed, a carbonate, a gelating agent gelated by calcium ion and a water-insoluble or poorly soluble calcium salt form(s) a granulated material, and further at least a thickener and water form a viscous material (hereinafter, the composition for preparing a carbon dioxide external agent [1-2]). In addition, a granular material in the present invention means solid material such as powder, fine granule, granule, crystal and the like or a mixture of these solid materials.

The composition for preparing a carbon dioxide external agent [1-1] above is comprised of a granular material, which was prepared in advance using an usual technique, comprising a substance generating an acid after being hydrolyzed, a carbonate and a thickener (hereinafter, the granular material [A]) and water. The granular material [A] is mixed with water to provide the carbon dioxide external agent at use.

The granular material [A] and water are kept without contact before use and the carbon dioxide external agent is easily obtained when the granular material [A] and water are mixed at use. The granular material [A] can be easily prepared when a substance generating an acid, a carbonate and a thickener are simply mixed for example. It is preferable that the substance generating an acid, the carbonate and the thickener are mixed as uniformly as possible.

A dispersant is preferably included in the granular material [A] so that the thickener is dissolved or uniformly dispersed in water when it is mixed with water by forming less so-called "DAMA" or "MAMAKO". The term "DAMA" or "MAMAKO" means an aggregate including no water inside, which is formed by aggregation of the solid thickener, and whose surface is covered with a dissolved or swelled viscous substance when the thickener is mixed with water.

There are no particular limitations on the dispersant above, so long as it is easily soluble in water, chemically stable and can be used in a form of granule, and examples thereof include a starch derivative such as pregelatinized starch, α-cyclodextrin and the like; a sugar derivative such as sugar, glucose, fructose, sucrose, lactose, xylitol, D-sorbitol, D-mannitol and the like; a polysaccharide such as pullulan, xanthan gum and the like; a cellulose derivative and salt thereof such as hydroxypropylcellulose, hydroxypropylmethylcellulose, carmellose calcium , carmellose sodium and the like; a synthetic polymer such as polyvinyl pyrrolidone; and urea etc; and one or more of these material(s) can be used.

Moreover, additives other than the above mentioned dispersant, which are commonly used in medicines or cosmetics, may be added so that the thickener is easily dissolved or uniformly dispersed in water.

In the composition for preparing a carbon dioxide external agent [1-2], it is preferable that the substance generating an acid after being hydrolyzed and/or the carbonate form(s) a granular material, and further at least the thickener and water form a viscous material, wherein the thickener is dissolved or uniformly dispersed in water.

If the thickener and water is not sufficiently mixed, the viscosity of the carbon dioxide external agent obtained is so deficient that the generated carbon dioxide leaks into the air and the amount of transdermally or transmucosally absorbed carbon dioxide may be decreased or the carbon dioxide external agent may droop down from the skin or mucosa. Therefore it is preferable that the thickener and water form a viscous material which is to be mixed with a granular material at use to prepare the carbon dioxide external agent having sufficient viscosity.

Examples of a combination between the granular material and the viscous material are as follows;
(1) a viscous material comprising a carbonate, a thickener and water (hereinafter, the viscous material [B]) and a granular material comprising a substance generating an acid after being hydrolyzed (hereinafter, the granular material [B]); and
(2) a viscous material comprising a thickener and water (hereinafter, the viscous material [C]) and a granular material comprising a substance generating an acid after being hydrolyzed and a carbonate (hereinafter, the granular material [C]).

In a case of the viscous material [B], wherein a thickener is blended in the viscous material combined with a carbonate, the neutral or alkaline thickener which is used in the composition for preparing a carbon dioxide external agent [1] described before is preferably used. The reason comes from the concern that a carbonate may react with the thickener to generate a carbon dioxide when preparing the viscous material if the thickener is acidic.

In a case of the viscous material [C], the same thickener used in the composition for preparing a carbon dioxide external preparation [1] (the neutral or alkaline, or acidic thickener above) can be used.

In the composition for preparing a carbon dioxide external agent [1], all the components are preferably mixed when the carbon dioxide external agent is at use in either case of the composition for preparing a carbon dioxide external agent [1-1] or the composition for preparing a carbon dioxide external agent [1-2].

When preparing the composition for preparing a carbon dioxide external agent [1-1] using a water-absorbing support material, the granular material [A] is preferably contained in the support material not being contacted with water before use. In such case, the carbon dioxide external agent can be used as a material for wound remedy or pack cosmetic, if only the water-absorbing support material is soaked in or moistened with water.

There are no particular limitations on the water-absorbing support material above, so long as it is possible to contain the granular material [A], having water absorbing capacity, and applicable to the skin. The water-absorbing support material may be any of a woven fabric, a nonwoven fabric, a sponge or the like, and can be selected as appropriate in accordance with the specific purpose, part of application and so on. Among them, a nonwoven fabric is light, and has excellent capacity of keeping the granular material and is thus particularly preferable.

The prepared carbon dioxide external agent is applied to the target part with thickness of 1 mm or more. The applied part may be covered with a plastic film etc. fixed by adhesive tape etc. so that the applied external agent is not moved from the original part. The carbon dioxide external agent is preferably applied for more than 5 minutes, and more preferably more than 10 minutes.

There is no limitation in the applying period, but it is preferable to exchange it with a new carbon dioxide external agent after about 24 hours. The external agent is preferably applied once a week or more and there is no limitation in applying times. Concentration of carbon dioxide is preferably over 300 ppm and more preferably over 1,000 ppm.

In addition, water of carbonated spring including an artificial carbonated spring, and carbon dioxide-containing water vapor also available. The carbon dioxide-containing water vapor means vapor such as natural carbon dioxide-containing water vapor which bursts from the ground, which is used for treating hemorrhoid etc. in place like Eastern Europe; and white smoke of carbon dioxide-containing water vapor which is generated by putting dry-ice in the air. A method of injecting gaseous carbon dioxide can also be available.

Of course, the present invention is not limited to these means for supplying carbon dioxide and any means which enable carbon dioxide to be absorbed into the targeted part in an amount required for preventing or treating fracture and a bone disease, or enhancing a bone growth.is acceptable. Among them, the above mentioned carbon dioxide external administration device is more preferable.

### EXAMPLES

The present invention is illustrated in more details by the following Examples. However, the present invention is not limited to these Examples.

### Example 1:

Bone growth promoting effect in rats (using a carbon dioxide external administration device as a means for absorbing a therapeutic agent for bone fracture, a bone growth promoting agent, or a therapeutic or prophylactic agent for bone diseases containing carbon dioxide as an active ingredient).

### Test Method

5-Week-old SD rats were used as test animals. A carbon dioxide absorption aid consisting of 93.77 % by weight of water, 5 % by weight of glycerin, 0.65 % by weight of carbomer, 0.15 % by weight of sodium alginate, 0.15 % by weight of sodium dihydrogen phosphate, 0.18 % by weight of sodium hydroxide, 0. 10 % by weight of methylparaben was applied twice a week to a hair-shaved right lower limb of a rat of the carbon dioxide absorption group (n = 6) in a thickness of 2 mm under anesthesia according to the patent document 7. The lower body of the rat was covered to seal with a bag having 4 cm width and 4 cm length and the top of the bag was bound tightly with a rubber band.

Then, the bag was filled with 100 % concentration of carbon dioxide through a vinyl tube from a carbon dioxide bomb in order to allow carbon dioxide to be absorbed for 10 minutes. The control group (n = 3) was subjected to only the anesthetic procedure. The above mentioned procedures were continued for 3 months. After 3 months, the tibias of both legs were collected from all rats and the length of them was measured by X-ray. After decalcification of the tibia, it was embedded by paraffin to prepare a tissue section. Auxotonic gristle area of the tissue section was stained with PCNA for staining proliferating cells in the area.

### Test Results

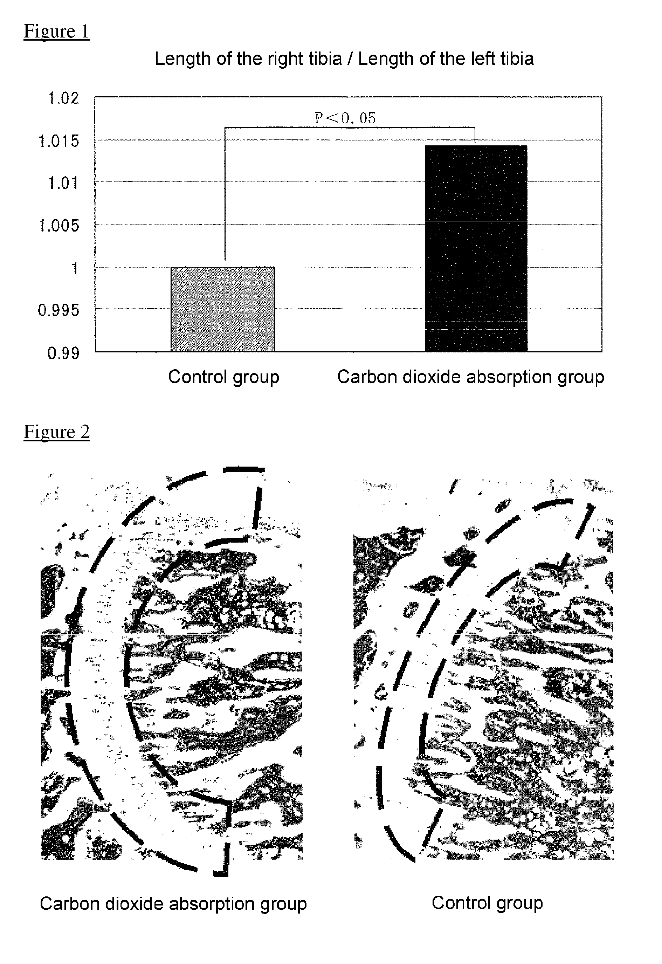

Upon comparing the ratio of the length between the right tibia and the left tibia, it was found as shown in FIG. 1 that the length of the right tibia of the carbon dioxide absorption group was statistically significantly longer than that of the control group (P < 0.05).

As shown in FIG. 2, the number of proliferating cells of the carbon dioxide absorption group was larger than that of the control group.

It is evident from the results above that a therapeutic agent for bone fracture, a bone growth promoting agent, or a therapeutic or prophylactic agent for bone diseases of the present invention containing carbon dioxide as an active ingredient, clearly has a bone growth promoting effect.

### Example 2:

Bone growth promoting effect in exercise-loaded rats (using a carbon dioxide external administration device as a means for absorbing a therapeutic agent for bone fracture, a bone growth promoting agent, or a therapeutic or prophylactic agent for bone diseases containing carbon dioxide as an active ingredient).

### Test Method

5-Week-old Wister rats were matched based on their body weight and were divided into two groups (a control group: n = 8; an exercise-loaded group: n = 16). The running training for 30 minutes per day using an activity wheel was given to all rats for five continuous days. The exercise-loaded rats were matched based on the running distance on the fourth and fifth day as well as the body weight on the fifth day and were divided into two groups (a training group: n = 8; a carbon dioxide absorption group: n = 8). After shaving the lower body hair of all rats, the running training for 30 minutes per day using an activity wheel was given to the training group and the carbon dioxide absorption group.

This training was done five times a week for 4 weeks. After training using the activity wheel, the carbon dioxide group was subjected to ether anesthesia, and then the same carbon dioxide absorption procedure as Example 1 was done to the both lower limbs of the rats for 10 minutes. After 2 days from the completion of the 4 weeks test, all rats were euthanized. The tibias of both legs were collected from the rats, and then the entire length of the tibias was measured by X-ray. After decalcification of the tibia, it was embedded by paraffin to prepare a tissue section. An auxotonic gristle plate of the tissue section was stained with Safranine O and its length was measured.

### Test Results

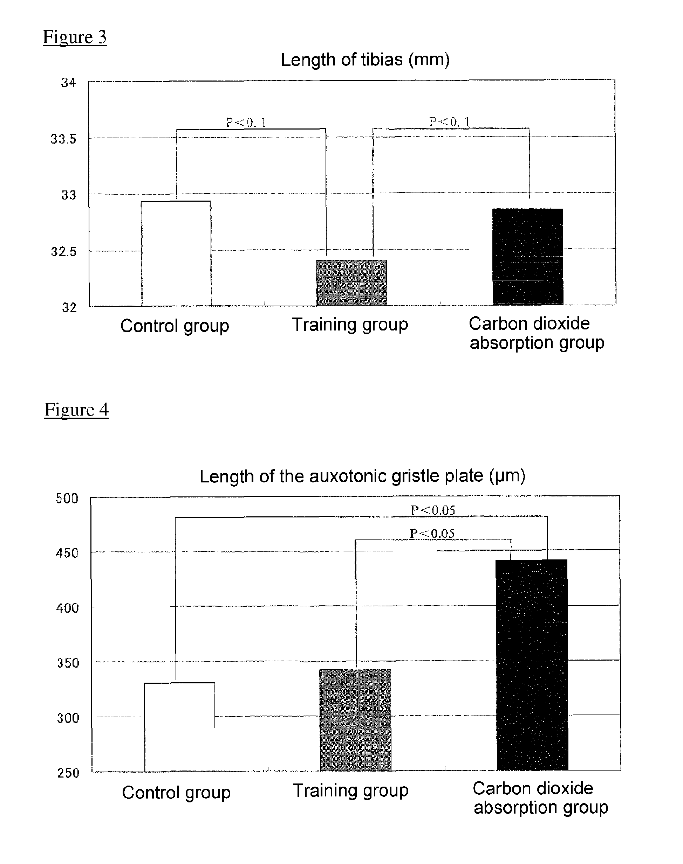

As shown in FIG. 3, the length of tibias of the training group was statistically significantly shorter than that of the control group which did not exercise (P < 0.1). The length of tibias of the carbon dioxide absorption group was statistically significantly longer than that of the training group (P < 0.1). Usually, the muscle development by exercise results in the prevention of tibia growth. However, the tibia growth was not prevented by exercise when a rat has been subjected to the carbon dioxide absorption. Therefore, it was evident that the carbon dioxide absorption promoted the bone growth.

As shown in FIG. 4, the length of the auxotonic gristle plate of the carbon dioxide absorption group was statistically significantly longer than that of the training group and the control group (P < 0.5).

It is evident from the results above that a therapeutic agent for bone fracture, a bone growth promoting agent, or a therapeutic or prophylactic agent for bone diseases of the present invention containing carbon dioxide as an active ingredient, clearly has a bone growth promoting effect.

### Example 3:

Bone fracture healing promoting effect in rats (using a carbon dioxide external administration device as a means for absorbing a therapeutic agent for bone fracture, a bone growth promoting agent, or a therapeutic or prophylactic agent for bone diseases containing carbon dioxide as an active ingredient)

### Test Method

According to the literature (Kokubo, T et al., J Orthop Res. 2003 May; 21(3): 503-510), a simple fracture was made in a femur of 12-week-old SD rats (n = 23). The rats were matched based on their body weight and were divided into two groups (a control group: n = 11; a carbon dioxide absorption group: n = 12). In the same procedures as Example 1 except for adopting 20 minutes of the carbon dioxide absorption time, carbon dioxide absorption into the fractured limb was conducted.

After making a fracture, X-ray images of the femur were taken for 2 rats of each group at the first week, 3 rats of each group at the second week, and 6 rats of the control group and the carbon dioxide absorption group at the third week. After the completion of the test, all rats were euthanized and the femurs were collected from the rats.

### Test Results

After making a fracture, it was not healed at the first and second week in both groups. At the third week, the fracture of 1/6 rats of the control group and 6/7 rats of the carbon dioxide group were completely healed.

It is evident from the above results that a therapeutic agent for bone fracture, a bone growth promoting agent, or a therapeutic or prophylactic agent for bone diseases of the present invention containing carbon dioxide as an active ingredient, clearly has a bone growth promoting effect.

### INDUSTRIAL APPLICABILITY

A therapeutic agent for bone fracture, a bone growth promoting agent, or a therapeutic or prophylactic agent for bone diseases of the present invention containing carbon dioxide as an active ingredient, is convenient to use with low cost and can shorten the period of time of a fracture healing with preventing pseudarthrosis and the like. The agents of the present invention can also prevent and treat bone diseases such as osteoporosis and rheumatoid arthritis. In addition, when used to a growing children or animals, the agents of the present invention can promote bone growth to increase body height and make bones less likely to cause fracture.

## Claims

1. A therapeutic agent for bone fracture, a bone growth promoting agent, or a therapeutic or prophylactic agent for bone diseases containing carbon dioxide as an active ingredient.

2. The therapeutic agent for bone fracture, a bone growth promoting agent, or a therapeutic or prophylactic agent for bone diseases according to Claim 1, wherein carbon dioxide is administered locally.

3. The therapeutic agent for bone fracture, a bone growth promoting agent, or a therapeutic or prophylactic agent for bone diseases according to Claim 2, wherein carbon dioxide is administered transdermally through the skin near the target site.

4. The therapeutic agent for bone fracture, a bone growth promoting agent, or a therapeutic or prophylactic agent for bone diseases according to Claim 2, wherein carbon dioxide is administered transdermally through the skin far from the target site.
